(19) Europäisches Patentamt / European Patent Office / Office européen des brevets

(11) **EP 4 473 935 B1**

(12) **EUROPEAN PATENT SPECIFICATION**

(45) Date of publication and mention
of the grant of the patent:
**06.05.2026 Bulletin 2026/19**

(21) Application number: **23770747.6**

(22) Date of filing: **13.03.2023**

(51) International Patent Classification (IPC):
*A61C 5/70* (2017.01)    *A61C 13/00* (2006.01)
*A61C 13/083* (2006.01)    *B33Y 10/00* (2015.01)
*B33Y 30/00* (2015.01)    *B33Y 80/00* (2015.01)

(52) Cooperative Patent Classification (CPC):
**B33Y 10/00; A61C 5/70; A61C 13/00;
A61C 13/0022; A61C 13/083; B33Y 30/00;
B33Y 80/00**

(86) International application number:
**PCT/JP2023/009694**

(87) International publication number:
**WO 2023/176792 (21.09.2023 Gazette 2023/38)**

(54) **MILL BLANK FOR DENTAL CUTTING AND METHOD FOR PRODUCING SAME**

FRÄSROHLING ZUM DENTALSCHNEIDEN UND VERFAHREN ZUR HERSTELLUNG DAVON

ÉBAUCHE DE FRAISE POUR APPAREIL DE COUPE DENTAIRE ET SON PROCÉDÉ DE PRODUCTION

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **15.03.2022 JP 2022040350**

(43) Date of publication of application:
**11.12.2024 Bulletin 2024/50**

(73) Proprietor: **Tokuyama Dental Corporation
Tokyo 110-0016 (JP)**

(72) Inventors:
• **NAGASAWA, Yuko
Tokyo 110-0016 (JP)**

• **IIDA, Shuntaro
Tokyo 110-0016 (JP)**

(74) Representative: **Dr. Schön, Neymeyr & Partner
Patentanwälte mbB
Bavariaring 26
80336 Munich (DE)**

(56) References cited:
WO-A1-2018/074605    WO-A1-2018/074605
JP-A- 2017 113 224    JP-A- 2018 533 431
JP-A- 2018 533 431    US-A1- 2018 325 635
US-A1- 2019 247 168

## Description

{Technical Field}

The present invention relates to a mill blank for dental cutting and a method for producing the same.

{Background Art}

Typically, a dental prosthesis used for dental treatment is molded from a metal material such as gold, silver, titanium, or a palladium alloy by casting, is shaped through the working of a nonmetal material such as ceramic or a hybrid resin (hereinafter, sometimes abbreviated as "HR"), or is formed using a metal material for forming a core part and using a nonmetal material for forming a surface layer part such as an anterior part and so on. Note that the hybrid resin means a composite material in which an inorganic filling material (hereinafter, sometimes referred to also as an "inorganic filler") is dispersed with a high density in a resin matrix, and is typically obtained by polymerization-curing a polymerization-curable composition in paste form containing a polymerizable monomer, an inorganic filler, and a polymerization initiator, by pressurizing and heating or the like.

The demand for a dental prosthesis entirely formed of a nonmetal material is rapidly increasing because it has the merits of not involving a risk of metal allergy and being aesthetically excellent and in addition, its working has become easy owing to the recent development of digital image technology, computer processing technology, and the like. A CAD/CAM system, as is disclosed in, for example, PTL1, has come into wide use that forms a dental prosthesis by cutting a blank for dental cutting made of a nonmetal material based on a photographed image of an oral cavity using a CAD/CAM device that is based on the techniques of CAD (Computer Aided Design) and CAM (Computer Aided Manufacturing). The blank for dental cutting here means an object to be cut (also called a mill blank) made attachable to a cutting machine in the CAD/CAM system, and typically has a section to be cut and a holding section such as a holding pin with which the section to be cut can be attached to the cutting machine. As the section to be cut, a (solid) block formed in a rectangular parallelepiped shape or a columnar shape, a (solid) disk formed in a plate shape or a disk shape, or the like is generally known, and a section to be cut formed of a hybrid resin (HR) is also known. Note that, in the present invention, a mill blank, including one in the form in which a section to be cut made of HR is integrated with a holding section, is called a HR-based mill blank for dental cutting or a HR-based mill blank.

Incidentally, in recent years, treatment for tooth crown and dentition restoration has been increasingly facing scenes where a dental prosthesis is required to have aesthetic properties such as color tone and appearance identical or close to those of natural tissues. To attain such aesthetic properties, the production not from a HR-based mill blank having one color tone but from a HR-based mill blank with a multilayer structure in which a plurality of layers with different color tones are integrated has been in practice, and for example, PTL2 describes a method for producing a HR-based mill blank with a multilayer structure, using fluid pastes, which are HR raw materials, and a plurality of formwork units. It describes that, according to this method, it is possible to produce the blank for dental cutting with the multilayer structure by filling the fluid pastes (curable compositions) with different color tones in the respective formwork units and stacking them, and even in a case where low-fluidity pastes which are usually difficult to pour into formworks are used, it is possible to produce a blank for dental cutting with a multilayer structure having a plurality of color tones and constitutions while reducing the occurrence of defects such as a disordered interface between the pastes and voids in the pastes.

Further, as a tooth mimicking technique different from the above-described configuration of stacking the layers, PTL3 proposes a HR-based mill blank whose color tone continuously varies as it goes from one end to the other end of the blank and a method for producing the same. Specifically, PTL3 discloses a method that, when filling a mixture of two types of fluid pastes (curable compositions) (which are HR raw materials) with different color tones from one corner to the opposite corner of a mold (for example, from a lower corner to an upper opposite corner of the mold) using an extruder such as a dispenser, continuously varies a mixture ratio of the two pastes in the mixture and cures the filled paste, thereby producing a mill blank for dental cutting (HR-based mill blank) having the above-described gradation structure (where color tone continuously varies). Further prior art is exemplified by PTL6.

{Citation List}

{Patent Literature}

{PTL 1} JP 2016-535610 A
{PTL 2} JP 2020-151339 A
{PTL 3} Specification of US Patent Application Publication No. 2019/247168

EP 4 473 935 B1

{PTL 4} JP 6285909
{PTL5} JP 6349480
{PTL6} WO 2018/074605 A1

{Summary of Invention}

{Technical Problem}

According to the method for producing the HR-based mill blank disclosed in PTL3 (hereinafter, referred to also as the production method of PTL3), it is possible to solve the problem of the discontinuity which occurs in a layer faying surface if the layered structure is employed. However, in a case where the aforesaid (paste) filling is performed while the mixture ratio is continuously varied at a predetermined rate, to produce the HR-based mill blank, the color tone becomes slightly different place by place even in a transverse surface having the same height, and accordingly, at some position where cutting is performed, the color tone may subtly deviate from an intended color tone. Further, in a case where a complete veneer crown (crown) is fabricated using the HR-based mill blank produced in the above-described manner, its color tone transition from a tooth cervix part to an incisal edge part tends to present an appearance lacking a three-dimensional effect when it adjoins with and is compared with an untreated natural tooth. Further, in a case where a plurality of prostheses are produced and different parts are treated at the same time, because of the size of some part to be treated, only part of the section to be cut may be used, for example, a partial veneer crown with a short crown height may be fabricated, and it has been found that in this case, there may occur unnatural gradation in the restored tooth.

Under such circumstances, the present invention has an object to provide a mill blank for dental cutting from which it is possible to fabricate a dental prosthesis that can more faithfully reproduce the gradation of a natural tooth and further makes it possible to obtain a natural three-dimensional effect after treatment without causing the aforesaid problems, and a method for producing the same.

{Solution to Problem}

The invention is as defined in the appended claims. The present invention solves the aforesaid problems, and a first aspect of the present invention is a mill blank for dental cutting including a section to be cut that has a shape of a columnar body whose bottom surface and upper surface are in a substantially identical shape and that is formed of a hybrid resin, the section to be cut having a gradation structure in which color appearance changes in stages in a height direction, where the height direction is a direction from the bottom surface of the columnar body being a base end toward the upper surface which is a tip,

wherein, in the gradation structure, a region having one color appearance forms each of "color units" into which the columnar body is divided in the height direction along a plane horizontal to the bottom surface and that each have a predetermined thickness in the height direction, and five to nine "color units" different in the color appearance are continuously arranged in the height direction, and

wherein, when the five to nine "color units" are each assigned a unit number: n (where n is a natural number of 1 to m) out of 1 to m (where m is a natural number representing the number of the units and is within a range of 5 to 9) to be denoted by a "color unit n" such that a value of the number increases in order from the tip side toward the base end side, and a color of the "color unit 1" located at the tip is represented by a "tip region color: A" and a color of the "color unit m" located at the base end is represented by a "base end region color: B",

a color difference: $\Delta E_{AB}$ between the tip region color: A and the base end region color: B is 5 to 20, and colors of the "color unit 2" to the "color unit m-1" are all intermediate colors: C between the colors A and B and change from an intermediate color close to A to an intermediate color close to B in stages from the "color unit 2" toward the "color unit m-1", and a color difference: $\Delta E_C$ between the colors of the adjacent units is 1 to 7.

Preferably, in the mill blank for dental cutting of the above aspect (hereinafter, referred to also as the "mill blank of the present invention"), the columnar body is formed of a single layer not having a laminate interface. Further, with the thickness in the height direction of each of the "color units" being defined as a height of each of the "color units", the heights of the "color unit 1" and the "color unit m" are each 15 to 35% of a whole height of the columnar body, and at this time, the height of the "color unit 1" and the height of the "color unit m" are optionally different.

A second aspect of the present invention is a method for producing the mill blank of the present invention, the method including: a raw material preparation step of preparing a hybrid resin raw material composition in paste form containing a polymerizable monomer, an inorganic filler, a polymerization initiator, and a coloring agent; a filling step of filling the hybrid resin raw material composition in a mold corresponding to the shape of the section to be cut; and a curing step of curing the hybrid resin raw material composition filled in the mold,

3

wherein the raw material preparation step prepares, as the hybrid resin raw material composition, a "color unit 1" raw material composition A giving a hybrid resin of the color: A, a "color unit m" raw material composition B giving a hybrid resin of the color: B, and (m-2) types of intermediate region hybrid resin raw material compositions that have constitutions corresponding to mixtures of the "color unit 1" raw material composition A and the "color unit m" raw material composition B and give the color appearances of the "color unit 2" to the "color unit m-1",

wherein the filling step includes:

a tip region filling step of filling, in the mold, a necessary amount of the "color unit 1" raw material composition A in order to form the "color unit 1";

a base end region filling step of filling, in the mold, a necessary amount of the "color unit m" raw material composition B in order to form the "color unit m"; and

an intermediate region filling step of filling, in the mold, necessary amounts of the intermediate region hybrid resin raw material compositions in order to form the "color unit 2" to the "color unit m-1", and

wherein the filling step is performed in either pattern out of:

(1) a pattern 1 in which the tip region filling step, the intermediate region filling step, and the base end region filling step are sequentially performed in the order mentioned; and

(2) a pattern 2 in which the base end region filling step, the intermediate region filling step, and the tip region filling step are sequentially performed in the order mentioned.

In the production method of the above aspect (hereinafter, referred to also as the "production method of the present invention"), preferably, the intermediate region filling step mixes the "color unit 1" raw material composition A and the "color unit m" raw material composition B with a mixture ratio of A and B being varied, to prepare the (m-2) types of intermediate region hybrid resin raw material compositions giving the colors of the "color unit 2" to the "color unit m-1", and in the pattern 1, the intermediate region hybrid resin raw material compositions are sequentially prepared in the order from the "color unit 2" raw material composition to the "color unit m-1" raw material composition, and the intermediate region hybrid resin raw material compositions in the amounts necessary for forming the "color units" are filled in the mold, and in the pattern 2, the intermediate region hybrid resin raw material compositions are sequentially prepared in the order from "the color unit m-1" raw material composition to the "color unit 2" raw material composition, and the intermediate region hybrid resin raw material compositions in the amounts necessary for forming the "color units" are filled in the mold while the raw material compositions are changed.

Further, preferably, the intermediate region filling step performs the preparation and the filling of each of the "color unit" raw material compositions simultaneously, using tanks capable of separately holding two types of fluids and a discharge device capable of uniformly mixing the two types of fluids held in the tanks, at a desired ratio and discharging a mixture from a discharge port.

In the above-described preferable mode, in the pattern 1, when the raw material composition A and the raw material composition B are mixed in the intermediate region filling step after the tip region filling step is performed by discharging only the raw material composition A using the discharge device, the intermediate region filling step is performed by preparing the intermediate region hybrid resin raw material compositions for the "color units" while reducing a ratio of the raw material composition A occupying the obtained mixtures in stages, and sequentially discharging the intermediate region hybrid resin raw material compositions, and thereafter the base end region filling step is performed by discharging only the raw material composition B, and

in the pattern 2, when the raw material composition A and the raw material composition B are mixed in the intermediate region filling step after the base end region filling step is performed by discharging only the raw material composition B using the discharge device, the intermediate region filling step is performed by preparing the intermediate region hybrid resin raw material compositions for the "color units" while reducing a ratio of the raw material composition B occupying the obtained mixtures in stages and sequentially discharging the intermediate region hybrid resin raw material compositions, and thereafter the tip region filling step is performed by discharging only the composition A.

Further, in the above-described mode, preferably, when a position in the mold is expressed by a coordinate system whose origin is a given point on a bottom surface in the mold and whose x-axis and y-axis represent a lateral direction and a longitudinal direction of the bottom surface respectively and whose z-axis represents a height direction, and (m-1) pieces of z-coordinates corresponding to heights of boundaries between m pieces of the "color units" that are to be formed in the mold are denoted by $z_1$ to $z_{m-1}$ from the bottom surface side,

the filling of each of the types of "color unit" raw material compositions in the mold in the filling step is performed using the discharge device capable of freely controlling a position of the discharge port in a front-rear direction, a left-right direction, and the height direction, by:

(i) setting the position of the discharge port when the filling starts, at the origin;

(ii) moving the position of the discharge port in the x-axis direction and/or the y-axis direction to scan a whole region on the bottom surface once while discharging the hybrid resin raw material composition at a predetermined flow rate from the discharge port, to form a mold lowest filling region having a uniform thickness corresponding to the one scanning;

(iii) moving the position of the discharge port in the z-axis direction to a height of an upper surface of the mold lowest filling region or higher after finishing the scanning in (ii);

(iv) moving the position of the discharge port in the x-axis direction and/or the y-axis direction to scan a whole region on the upper surface of the mold lowest filling region as a foundation once while discharging the hybrid resin raw material composition at a predetermined flow rate in the same manner as in the (ii), to form a grown filling region resulting from addition of a uniform thickness corresponding to the one scanning; and

(v) repeating the operations (iii) and (iv) continuously with the grown filling region as the foundation, and

the varying of the ratio in the hybrid resin raw material composition to be discharged is performed in the operations (ii) to (iv) such that, at an instant when the thickness of the formed grown filling region becomes equal or substantially equal to each of the heights zi to $z_{m-1}$, the raw material composition for the next "color unit" is discharged from the discharge port.

{Advantageous Effects of Invention}

According to the mill blank of the present invention, it is possible to produce a dental prosthesis that can more faithfully reproduce the gradation of a natural tooth and further makes it possible to obtain a natural three-dimensional effect after treatment, irrespective of the shape of the dental prosthesis to be produced (for example, in a case where a crown is to be produced, irrespective of whether it is a complete veneer crown or a partial veneer crown having a short crown height). Further, in the case where the mill blank of the present invention is not a multilayer body but has a single-layer structure whose color appearance changes in the height direction in stages, no gradation disorder accompanying the disorder on a laminate interface occurs, nor does a trouble such as a fracture along the laminate interface occur during cutting.

Further, according to the production method of the present invention, it is possible to efficiently produce the mill blank of the present invention, in particular, the mill blank having the aforesaid single-layer structure.

{Brief Description of Drawings}

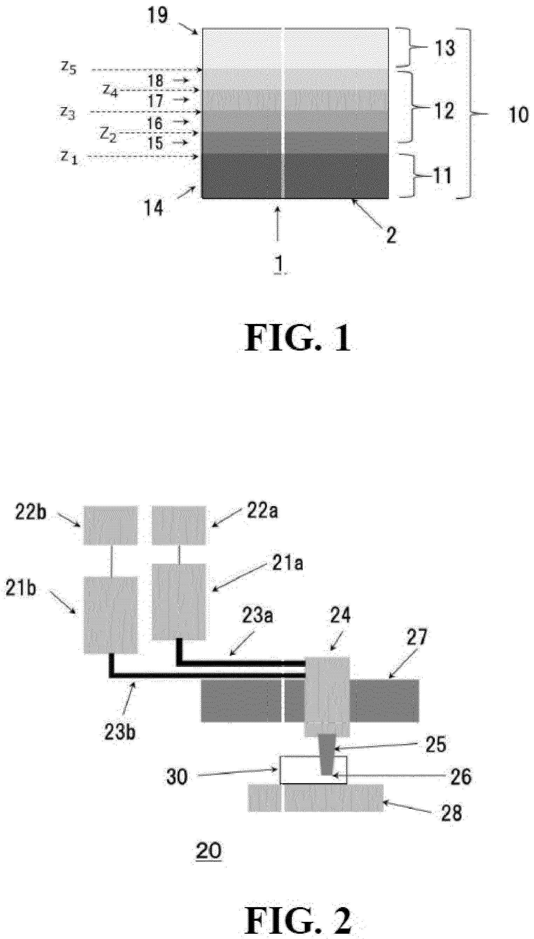

{Fig. 1} This drawing is a view schematically illustrating a representative cross section of a section to be cut in the mill blank of the present invention. Note that the vertical direction in the drawing is a height direction (z-axis direction).

{Fig. 2} This drawing is a schematic view to explain the structure of a discharge device suitably usable in the production method of the present invention.

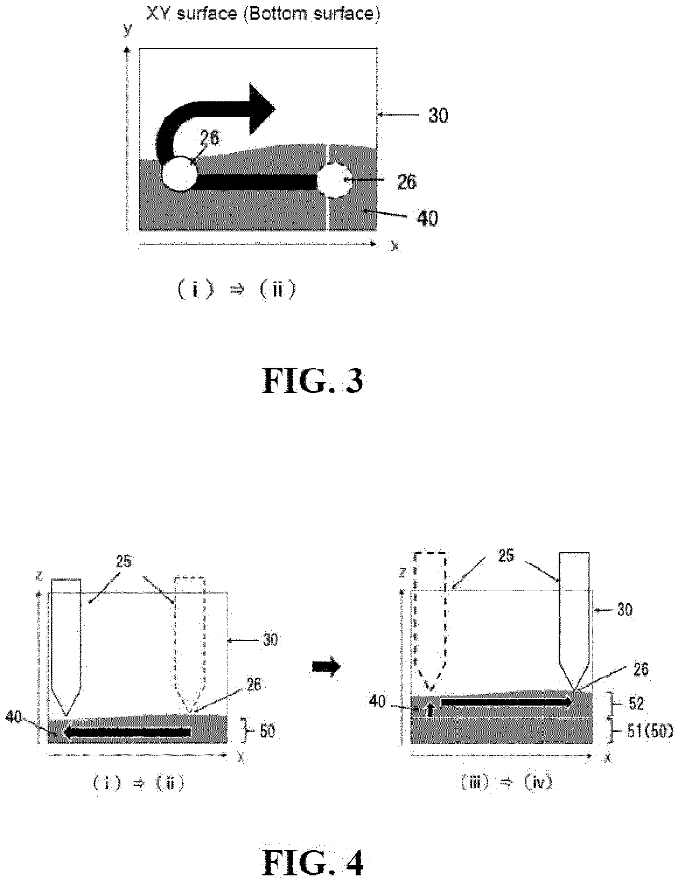

{Fig. 3} This drawing is a view schematically illustrating a state (change state) on a bottom surface at the time of forming a mold lowest filling region when a filling step in the production method of the present invention is performed using the discharge device illustrated in Fig. 2. Note that the arrow in the drawing indicates a movement direction of a nozzle discharge port.

{Fig. 4} This drawing is a view schematically illustrating a state (change state) in the mold seen from its major surface direction, at the time of forming the mold lowest filling region (left drawing) and at the time of forming a grown filling region thereon (right drawing) when the filling step in the production method of the present invention is performed using the discharge device illustrated in Fig. 2. Note that the arrows in the drawing indicate movement directions of the nozzle discharge port.

{Description of Embodiments}

According to the production method of PTL3, it is possible to give a continuous shade gradient to the section to be cut formed of HR, but as described above, in the case where the paste is filled while the mixture ratio of the two types of pastes is varied at a predetermined rate, it is difficult to obtain a blank from which a dental prosthesis having a color tone harmonic with a natural tooth and a three-dimensional effect can be produced. A conceivable reason for this is that the colors of a dentine and an enamel covering this reflect their internal structures, and the color tone of the natural tooth is visually recognized as the result of the fusion of these colors, but not only the original color tones of the dentine and the enamel greatly differ but also their thickness ratio does not always change continuously and uniformly from the tooth cervix part side toward the incisal edge side.

In the mill blank of the present invention, on the other hand, to reproduce the discontinuous color change of a natural tooth from a tooth cervix part toward an incisal edge part, in the gradation in a section to be cut, color tone does not change continuously but changes in the height direction discontinuously (in stages), and "color units" which are regions having respective colors are formed such that those at ends are large in thickness and those at a middle region are small in thickness, and further, in the middle region, the color change in the height direction in stages is set fine (a color difference: $\Delta E$ between adjacent ones in three to seven color units is set to 1 to 5). Such a configuration produces optical illusion effects such as Chevreul illusion and Mach bands (the vicinity of an interface where a uniform layer is in contact with another layer is visually exaggerated), and person's recognition of partial exaggeration is thought to be a reason why it is possible to reproduce the discontinuous appearance seen in the natural tooth.

The mill blank of the present invention and the production method of the present invention will be hereinafter described in detail.

1. Mill Blank of Present Invention

The mill blank of the present invention is a mill blank for dental cutting including a section to be cut that has the shape of a columnar body whose bottom surface and upper surface are in a substantially identical shape and that is formed of a hybrid resin. It is characterized in that the section to be cut has a special gradation structure.

1-1. Regarding the material and shape of the section to be cut

The mill blank of the present invention has the section to be cut formed of the hybrid resin (HR), and the section to be cut has the shape of the columnar body whose bottom surface and upper surface are in the substantially identical shape. The mill blank having the section to be cut with such a material and a shape is common as a HR-based mill blank, and the mill blank of the present invention does not have any particular difference in the basic material and shape from the common HR-based mill blank.

For example, as for the shape, a columnar body or a disk-shaped body that can be set in a commercially available dental CAD/CAM system and that has a size large enough to be worked into one tooth or a plurality of teeth can be used without any restriction. Examples of a preferable size include: a 40 mm × 20 mm × 15 mm rectangular prismatic shape appropriate for forming one-tooth missing bridge; a prismatic shape with 15 mm × 8 mm × 8 mm or 15 mm × 10 mm × 10 mm appropriate for forming an inlay or an onlay; a prismatic shape with 20 mm × 18 mm × 14 mm, 15 mm × 12 mm × 10 mm, 15 mm × 12 mm × 6.5 mm, 18 mm × 14 mm × 12 mm, 18 mm × 14 mm × 16 mm, 15 mm × 14.5 mm × 14.5 mm, 18 mm × 14.5 mm × 14.5 mm, or 18 mm × 14.5 mm × 16 mm appropriate for forming a full crown; and a disk shape with a 90 to 100 mm diameter and a 10 to 28 mm thickness appropriate for forming a long-span bridge or a denture plate.

In consideration of the production by the production method of the present invention, a large-volume blank like a disk necessitates a longer time for filling the HR raw material composition at the time of the production and in addition, is more difficult to obtain layer flatness than a smaller blank, and thus a columnar shape or a prismatic shape is preferable. On the other hand, in a small workpiece, it is difficult to perceive a color tone difference. Therefore, a blank with a certain degree of thickness and size leads to more efficient and lower-cost production. The section to be cut, if having a 6 to 25 mm height, can be adapted to teeth with a typical size. A small height makes it difficult to obtain a later-described color tone change effect, and thus the height is preferably 8 mm or more, and is most preferably 10 mm or more. A large height facilitates the adaptation to various tooth crown shapes and makes it easy to clearly express a color tone difference from the middle, but a size larger than 25 mm leads to an increase of vainly cut parts.

Further, as for the material (HR) as well, there is no special difference from conventional HR except that the kind and compounding amount of a dying component such as a pigment compounded for achieving the specific gradation structure are partly different. Note that raw materials of HR will be detailed in the description regarding the production method of the present invention.

1-2. Regarding the gradation structure

The section to be cut of the mill blank of the present invention has the gradation structure in which color appearance changes in stages in a height direction, where the height direction is a direction from the bottom surface of the columnar body being a base end toward the upper surface which is a tip, and the gradation structure needs to satisfy all the following conditions (1) to (3) to achieve the above-described effect (hereinafter, the gradation structure satisfying these conditions will be referred to also as the "gradation structure of the present invention").

(1) A region having one color appearance forms each of "color units" into which the columnar body is divided in the height direction along a plane horizontal to the bottom surface and that each have a "predetermined thickness in the height direction" (hereinafter, also referred to simply as "height" or "thickness"), and five to nine "color units" different in the color appearance are continuously arranged in the height direction. It should be noted that the aforesaid "plane horizontal to the bottom surface" does not necessarily have to be a flat surface and may be a curved surface (for example, a curved surface like a wavy surface) having unavoidable or intentionally formed minute projections and indentations {with a level difference being, for example, 1.0 mm or less and preferably 0.5 mm or less} from a reference plane being the "plane horizontal to the bottom surface".

(2) When the five to nine "color units" are each assigned a unit number: n (where n is a natural number of 1 to m) out of 1 to m (where m is a natural number representing the number of the units and is within a range of 5 to 9) to be denoted by a "color unit n" such that a value of the number increases in order from the tip side toward the base end side, and a color of the "color unit 1" located at the tip is represented by a "tip region color: A" and a color of the "color unit m" located at the base end is represented by a "base end region color: B", a color difference: $\Delta E_{AB}$ between the tip region color: A and the base end region color: B is 5 to 20.

(3) Colors of the "color unit 2" to the "color unit m-1" are all intermediate colors: C between the colors A and B and change from an intermediate color close to A to an intermediate color close to B in stages from the "color unit 2" toward the "color unit m-1", and a color difference: $\Delta E_C$ between the colors of the adjacent units is 1 to 7.

Hereinafter, the gradation structure of the present invention will be described with reference to Fig. 1 schematically illustrating a vertical sectional view (taken along a plane vertical to the bottom surface 2) of a section to be cut 1 formed of a columnar body and having six "color units" (m = 6). The gradation structure 10 of the present invention illustrated in Fig. 1 has a "color unit 6" 14 including the bottom surface 2 and having the "base end region color: B", a "color unit 1" 19 including the upper surface and having the "tip region color: A", and four "color unit 2" 18 to "color unit 5" 15 present between them and having the intermediate colors: C between the colors A and B. However, the gradation structure of the present invention is not limited to that illustrated in Fig 1, and m representing the number of the "color units" may be 5 to 9 and is preferably 5 to 7.

The "color units" mean the regions that each have one color appearance, and into which the columnar body is divided in the height direction along the plane horizontal to the bottom surface, and that each have a predetermined thickness as described in the above (1), but a boundary surface of the "color units" which are adjacent to each other due to the above-described production method need not be a strictly flat surface and may be one considered as a substantially flat plane. For example, it may be a curved surface (for example, a curved surface like a wavy surface) having minute projections and indentations {with a level difference being, for example, 1.0 mm or less, and preferably 0.5 mm or less} from a reference plane being the "plane horizontal to the bottom surface".

Further, the "color unit" is a region having one color appearance, and partial regions in the single "color unit" have an identical color appearance. Here, "identical" means that the color appearance is not intentionally changed, and it is allowed that in a very thin region at the boundary of the adjacent "color units", the color appearances of these "color units" are a mixed color of their color appearances, and the color appearance slightly varies due to an unavoidable constitution variation that occurs when two types of curable compositions that are raw materials of HR are mixed to prepare a curable composition for filling. For example, in the case where the curable composition to be the raw material of HR is continuously extruded and filled into the mold while its constitution is slightly varied "continuously" and thereafter is cured as in the method disclosed in PTL3, the color appearance also varies according to the constitution variation, and thus it is not possible to form the "color unit".

The section to be cut of the mill blank of the present invention may have a multilayer structure in which the "color units" each form one (independent) layer and such layers are stacked (which has a distinct laminate interface between layers), but preferably has a single-layer structure in which the "color units" are highly integrated because it does not have a laminate interface which may have disorder or defect and could cause peeling or the like. It is possible to obtain such a single-layer structure in the production method of the present invention by scanning a nozzle and continuously extruding the curable composition which is to be the raw material of HR forming each of the "color units", to fill it into the mold while slightly varying its constitution "in stages", using a filling device such as a two-liquid mixing dispenser as will be described later.

The "color unit 1" (19 in Fig. 1) located on the most tip side of the columnar body and the "color unit 6 (= m) (14 in Fig. 1) located on its most base end side, which form the section to be cut of the mill blank of the present invention, each preferably have a thickness (height) that is 15 to 35% and more preferably 20 to 30% of the whole thickness (height) of the columnar body. Here, the thickness (height) of the "unit 1" and the thickness (height) of the "unit m" may be different. If the "color unit 1" and the "color unit m" are thinner than the aforesaid lower limit value, it tends to be difficult to clearly reproduce an incisal edge and a cervix part of a tooth, leading to a difficulty in adaptation to worked objects with different shapes. On the other hand, if they are too thicker than the aforesaid upper limit, it is possible to clearly reproduce the incisal edge and the cervix part of the tooth, but the color tone change from the "color unit 2" (18 in Fig. 1) to the "color unit 5 (= m-1) (15 in Fig. 1) becomes sharp and unnatural.

The color difference: $\Delta E_{AB}$ between the "tip region color: A" and the "base end region color B" needs to be 5 to 20.

This is based on the ordinary requirement that one (A or B) of these should have a color tone corresponding to the color tone of the tip region of the tooth and the other (B or A) should have a color tone corresponding to the color tone of the cervix part of the tooth and its vicinity region. If $\Delta E_{AB}$ falls out of the **aforesaid** range, this is unnatural as a combination of the color tone of the tip region and the color tone of the cervix part and its vicinity region in one natural tooth.

Note that the color difference ($\Delta E$) is an index representing a degree of difference between two colors, and its value being larger means a larger difference between their color tones. The color difference: $\Delta E$ (sometimes also represented by $\Delta E^*$) in the present invention means $\Delta E$ determined regarding two color tones to be contrasted by the following formula using differences ($\Delta L^*$, $\Delta a^*$, and $\Delta b^*$) regarding numerical values ($L^*$ values, $a^*$ values, and $b^*$ values) in the coordinate axes in the $L^*a^*b^*$ color space (color system) where lightness is represented by $L^*$ and chromaticity indicating hue and saturation is represented by $a^*$ and $b^*$.

$$\Delta E = \{(\Delta L^*)^2 + (\Delta a^*)^2 + (\Delta b^*)^2\}^{1/2}$$

Further, the $L^*$ value, the $a^*$ value, and the $b^*$ value in this specification are determined by $L^*$, $a^*$, and $b^*$ obtained in a sample with a $1.0\pm0.1$ mm thickness by white-background spectral reflectance factor measurement by a spectrophotometer (for example, "TC-1800MKII" manufactured by Tokyo Denshoku, or the like). Specifically, in the case of a colorimeter "TC-1800MKII" manufactured by Tokyo Denshoku, the measurement conditions are as follows: a halogen lamp: 12 V 100 W, a measurement wavelength range 380 to 780 nm, reflected light 0° d method (JIS Z8722), measurement area 5 mm$\phi$, and white background (state where a standard white plate is laid on a cured body to shut off light). Note that the fabrication of the measurement samples and the measurement therein can be done by (i) a method of cutting out the vicinity of the center of each unit from the section to be cut along the direction parallel to the bottom surface to form a measurement test piece (where a transverse section of the single color unit is exposed), and subjecting them to the measurement by the colorimeter, (ii) a method of cutting out from the section to be cut along the direction perpendicular to the bottom surface to form a measurement test piece (where the vertical sections of all the color units are exposed, and subjecting it to the measurement by line-scanning in the direction from the tip to the base end, or (iii) in the case where the constitutions of the raw material compositions of HR forming the "color units" are known, a method of conducting the measurement by the colorimeter regarding samples that are fabricated using raw material compositions having these constitutions. In the case where the above method (i) is employed, the measurement is conducted regarding two front and rear surfaces of the sample, and average values of the results may be used as the $L^*a^*b^*$ values of the relevant "color unit". Further, in the case where the above method (ii) is employed, values measured in the vicinity of the center of each color unit may be used as the $L^*a^*b^*$ values of the relevant "color unit".

The color corresponding to the color tone of the tip region may be either A or B as desired but is A in the mode illustrated in Fig. 1. In the case where A is the color corresponding to the color tone of the tip region, A is typically selected from A1, A2, and A3, and B is typically selected from A3, A3.5, and A4 in terms of the shade in the 16-Colors Shade Guide of vita Co., Ltd. Further, by turning A into a color tone paler than A1 or turning B into a deeper color tone than A4, it is possible to increase color tone variations that can be created from the combination of A and B.

Further, in the gradation structure 10 of the present invention, in the "color unit 2" 18 to the "color unit 5 (= m-1)" 15, it is necessary that the colors of these units be all intermediate colors: C between the colors A and B and change from an intermediate color close to A to an intermediate color close to B in stages from the "color unit 2" toward the "color unit m-1", and a color difference: $\Delta E_C$ between the colors of the adjacent units be 1 to 5. Specifically, let a plurality of different colors belonging to the intermediate color: C between the colors A and B be $C_n$ (where n represents a natural number of 2 to m-1, and n with a smaller value means an intermediate color closer to A, and n with a larger value means an intermediate color closer to B), the colors of the "color unit 2" to the "color unit m-1" are $C_2$ to $C_{m-1}$ respectively, and the color difference: $\Delta E_C$ between the intermediate color $C_{n-1}$ and the intermediate color $C_n$ needs to be constantly 1 to 7, and is more preferably 1 to 5. Further, a color difference: $\Delta E_{AC2}$ between A and $C_2$ and a color difference $\Delta E_{BCm-1}$ between B and $C_{m-1}$ are also 1 to 7 and especially preferably 1 to 5. However, individual values of $\Delta E_C$, $\Delta E_{AC2}$, and $\Delta E_{BCm-1}$ may be different as long as they fall within the aforesaid range. For example, it is also possible to gradually increase or gradually decrease a variation width from the "color unit 1" to the "color unit m" or set it large in the middle part.

2. Production Method of Present Invention

The production method of the present invention is a method for producing the mill blank of the present invention and similarly to a conventionally typical method for producing a HR-based mill blank, obtains the section to be cut by preparing a HR raw material composition in paste form containing a polymerizable monomer, an inorganic filler, a polymerization initiator, and a coloring agent (raw material preparation step), filling it in a mold (filling step), and thereafter curing it (curing step), and is characterized in that the raw material preparation step prepares preferably two types of HR raw material compositions and the filling step employs a special filling method using these, thereby making it possible to efficiently produce the mill blank of the present invention. Hereinafter, the steps in the production method including what are not different from the conventionally typical method will be described.

2-1. The raw material preparation step

The raw material preparation step prepares, as the HR raw material composition in paste form containing the polymerizable monomer, the inorganic filler, the polymerization initiator, and the coloring agent, a "color unit 1" raw material composition A giving HR of the color: A and a "color unit m" raw material composition B giving HR of the color: B, and further prepares, as HR raw material compositions for forming the "color unit 2" to the "color unit m-1", (m-2) types of intermediate region HR raw material compositions having constitutions corresponding to mixtures of the raw material composition A and the raw material composition B and giving color appearances of the respective "color units". At this time, the (m-2) types of intermediate region HR raw material compositions may be prepared by any method as long as it is a method that makes the constitutions after the preparation become the constitutions corresponding to the mixtures of the raw material composition A and the raw material composition B, but are preferably prepared by directly mixing the "color unit 1" raw material composition A and the "color unit m" raw material composition B with a mixture ratio of these being varied, prepared by mixing two or more compositions which are raw materials of the "color unit 1" raw material composition A with the "color unit m" raw material composition B or two or more compositions which are its raw materials, with their mixture ratio being varied, or prepared by mixing the "color unit 1" raw material composition A or two or more compositions which are its raw materials with the "color unit m" raw material composition B, with their mixture ratio being varied, and are most preferably prepared by directly mixing the "color unit 1" raw material composition A and the "color unit m" raw material composition B with their mixture ratio being varied.

A cured body of the polymerizable monomer which is the raw material of the HR raw material composition in paste form is to be a resin matrix of HR. As the polymerizable monomer, a radical polymerizable monomer is suitably usable. The radical polymerizable monomer is not limited, and one appropriately selected from cationic polymerizable monomers such as a (meth)acrylic compound, epoxies, oxetanes, and so on is usable. For example, to obtain a dental polymerization-curable composition, a (meth)acrylic compound is preferably used. As required, a plurality of kinds may be used in combination.

The inorganic filler is a filling material that is added to increase the strength of the HR cured body, increase elasticity to reduce bending, increase abrasion resistance, inhibit polymerization shrinkage, and adjust fluidity. Any known inorganic filler used in curable compositions is usable. Silica-based composite oxide particles among inorganic particles facilitate adjusting a refractive index. Further, they are especially preferable because, owing to the presence of a large number of silanol groups on their particle surfaces, surface modification is easy using a silane coupling agent or the like. Particles of silica-zirconia, silica-titanium, silica-titanium-barium oxide, silica-titanium-zirconia, and the like are suitable because of their high X-ray contrast properties. Further, silica-titanium particles and silica-zirconia particles are the most preferable because they help obtain a cured body having higher abrasion resistance. The aforesaid inorganic filler may be compounded as organic-inorganic composite particles. A filling factor of the inorganic filler in HR is preferably 50 to 99 wt%, more preferably 60 to 95 wt%, and still more preferably 65 to 90 wt%. The filling factor of the inorganic filler is most preferably 70 wt% or more because, if the filling factor of the inorganic filler is low, physical properties are degraded though fluidity usually increases and thus the merit of facilitating the filling is obtained. On the other hand, as the filling factor is increased, a dental blank having excellent physical properties as a dental prosthesis is obtained, but if it is too high, the filling becomes difficult due to an influence of a low fluidity, and thus, the filling factor is most preferably 85 wt% or less.

The polymerization initiator is not limited as long as it has the function of polymerization-curing the polymerizable monomer. Polymerization methods of a curable composition include one using a reaction caused by light energy such as ultraviolet rays or visible rays (hereinafter, referred to as photopolymerization), one using a chemical reaction of peroxide and an accelerating agent, and one using heat energy (hereinafter, referred to as thermal polymerization), and any of these methods may be used. Photopolymerization or thermal polymerization is preferable because they allow the desired selection of when to cause the polymerization by externally given energy such as light or heat and their operation is simple, and thermal polymerization is especially preferable because it is not likely to cause uneven polymerization or the like due to light irradiation and its polymerization reaction can be uniform. Note that the polymerization initiator to which a photoacid generator in addition to a reductant compound is added may be used. These polymerization initiators are each sometimes used alone, but a mixture of two kinds or more thereof may be used. Further, a plurality of initiators whose polymerization methods are different can be combined. As a compounding amount of the polymerization initiator, an effective amount may be selected according to its intended use, but its ratio is typically 0.01 to 10 parts by mass, and more preferably, one whose ratio is 0.1 to 5 parts by mass to 100 parts by mass of the polymerizable monomer is used.

The coloring agent is compounded to adjust the color of the paste, and consequently the color of the hybrid resin which is its cured body. As the coloring agent, any coloring substance such as pigments and dyes conventionally used for dental application is usable without any restriction, and according to an intended color (A or B), a necessary amount of one of these or an appropriate combination of a plurality of these may be compounded.

A kneading method in the case where predetermined amounts of the aforesaid components are mixed to prepare the raw material composition A, the raw material composition B, and the compositions which are the raw materials of these is preferably one using a kneading device such as a planetary stirrer because this makes it possible to satisfy the aforesaid dispersion condition in a short time and facilitates scale-up production. From a viewpoint of the capability of continuous kneading, a single-screw kneading machine or a multi-screw kneading machine is also usable. Further, for defoaming, a defoaming method under a reduced pressure is preferably employed because it can remove air bubbles in a short time.

2-2. The filling step

The filling step includes: a tip region filling step of filling, in the mold, a necessary amount of the raw material composition A in order to form the "color unit 1"; a base end region filling step of filling, in the mold, a necessary amount of the "color unit m" raw material composition B in order to form the "color unit m"; and an intermediate region filling step of filling, in the mold, necessary amounts of the (m-2) types of intermediate region HR raw material compositions in order to form the "color unit 2" to the "color unit m-1".

These steps are performed in either pattern out of (1) a pattern 1 in which the tip region filling step, the intermediate region filling step, and the base end region filling step are sequentially performed in the order mentioned and (2) a pattern 2 in which the base end region filling step, the intermediate region filling step, and the tip region filling step are sequentially performed in the order mentioned.

At this time, preferably, the intermediate region filling step mixes the raw material composition A and the raw material composition B with their mixture ratio being varied, to prepare the (m-2) types of intermediate region HR raw material compositions giving the colors: of the "color unit 2" to the "color unit m-1". Preferably, in the pattern 1, the intermediate region HR raw material compositions are sequentially prepared in the order from the "color unit 2" raw material composition to the "color unit m-1" raw material composition, and these compositions in the amounts necessary for forming the "color units" are filled in the mold, and in the pattern 2, the intermediate region HR raw material compositions are sequentially prepared in the order from the "color unit m-1" raw material composition to the "color unit 2" raw material composition and these compositions in the amounts necessary for forming "color units" are filled in the mold while the raw material compositions are changed.

Note that, as the material of the mold used in the filling, metal, ceramic, resins, rubber, or the like is usable according to the intended use, and a material having heat resistance against a higher temperature than the temperature of the polymerization executed in the later-described curing step is preferably used. Examples of the material of the mold include SUS, high-speed tool steel, an aluminum alloy, polyethylene terephthalate (PET), polyethylene (PE), polypropylene (PP), polystyrene (PS), hard rubber, and silicone rubber. As required, the inner surface of the mold may be subjected to surface treatment such as adhesion prevention.

Incidentally, disorder on the boundary between the adjacent "color units", if occurs, may make it impossible to obtain the intended gradation structure after the cutting, and further, a defect on the boundary, if any, involves a risk of causing breakage starting therefrom. Therefore, the section to be cut is preferably a single-layer structure in which the "color units" are highly integrated without any laminate interface including disorder or defect being present between the adjacent "color units". It is possible to obtain the section to be cut having such a single-layer structure by the aforesaid method of scanning the nozzle and continuously extruding the composition to fill it into the mold in the filling step while slightly varying its constitution "in stages" using the filling device such as a two-liquid mixing dispenser (hereinafter, referred to also as the "continuous filling method"). Therefore, the filling device suitably used for obtaining the section to be cut with the single-layer structure and the continuous filling method using the filling device will be hereafter described.

2-3. The suitable filling device

For the abovementioned reasons, the filling of the HR raw material composition in the mold in the filling step is preferably performed using tanks capable of holding two types of fluids separately and a discharge device capable of uniformly mixing the plurality of types of fluids held in the tanks at a desired ratio to discharge the mixture from a discharge port. As such a discharge device, what is called a multi-liquid mixing dispenser, is suitably used. Fig. 2 is a schematic view illustrating the basic structure of a representative multi-liquid mixing dispenser (two-liquid mixing dispenser) 20 capable of mixing two fluids (liquid compositions), and it mixes, at a desired ratio, liquid compositions stored (held) in two "tank parts" 21a, 21b by a "mixer part" 24 having a mixing mechanism such as a static mixer or a mixer having a rotating mechanism and discharges the mixture from a discharge port 26 of a "discharge part" 25 such as a nozzle, thereby capable of filling it in the mold (shaping mold) held on a stage part. Specifically, upstream of the two "tank parts" 21a, 21b, "pressurizing/pressure-reducing parts" 22a, 22b are provided respectively, "pipe parts" 23a, 23b leading to the "mixer part" 24 are connected to downstream parts of the "tank parts" 21a, 21b, and a not-illustrated "control part" controls and drives the "pressurizing/-pressure-reducing parts" 22a, 22b independently so that the two types of liquid compositions are fed to the "mixer part" 24 at a controlled extrusion speed, and there, the two types of liquid compositions are uniformly mixed and the mixture is extruded (discharged) from the discharge port 26 of the "discharge part" 25. At this time, it is of course possible to discharge the single liquid composition as it is from the discharge part 25 (the discharge port 26 of the discharge nozzle) without mixing the liquid compositions in the "mixer part" 24. The multi (two)-liquid mixing dispenser illustrated in Fig. 2 further has an "arm part" 27 for holding the "mixer part" 24 and the "stage part" 28 where to hold and place the mold 30, and the not-illustrated "control part" is configured to be capable of freely controlling the positional relationship of the "discharge port 26 of the "discharge part" 25 relative to the mold 30 in the front-rear, left-right, and up-down directions by moving the "arm part" 27 and/or the "stage part" 28 back and forth and up and down (hereinafter, the multi-liquid mixing dispenser having such a structure will be referred to also as the "multi-liquid mixing dispenser with a position control mechanism"). As required, the multi-liquid mixing dispenser with the position control mechanism may further have a "heating part" (not illustrated) for temperature adjustment. In the multi-liquid mixing dispenser having such a basic structure, by increasing the number of sets each composed of the "pressurizing/pressure-reducing part", the "tank part", and the "pipe part" connected to the mixer part, it is possible to increase the number of liquid compositions to be mixed according to the number of the sets, and thus it is possible to set a combination of the types of pastes to be mixed in the mixer part and a mixture ratio thereof as desired. Such a multi-liquid mixing dispenser with the position control mechanism is generally used for applying a liquid material such as an adhesive in a predetermined pattern at the time of electronic device production (see, for example, PTLs 4 and 5), and customized ones are also commercially available.

The use of the multi-liquid mixing dispenser with the position control mechanism enables the simultaneous execution of the preparation of each of the HR raw material compositions in paste form for the "color unit 2" to the "color unit m-1" and its filling (into the mold). For example, in a case where one having the structure illustrated in Fig. 2 is used, if the pre-prepared raw material compositions A and B are stored in the two "tank parts" 21a, 21b, it is only necessary to discharge only the raw material composition A and fill it when the "color unit 1" is to be filled, to discharge only the raw material composition B and fill it when the "color unit m" is to be filled, to mix the raw material compositions A and B at mixture ratios such that intended color tones can be obtained and perform the filling when the "color unit 2" to the "color unit m-1" are to be filled. Further, for example, in a case where the filling for a section to be cut with a different color arrangement pattern where the tip region color is A' and the base end region color is B' is performed, pastes giving the color tones of A' and B' may be prepared and filled by performing the mixing of the raw material compositions A and B also at the time of the filling for the "color unit 1" and the "color unit m". Another possible configuration may be to use a device having three or more "tank parts", store, in the tanks, HR raw material compositions in paste form giving different color tones, and appropriately mix these to prepare pastes for the respective "color units" and fill them.

2-4. The filling method using the suitable filling device

The case where the filling step is performed using the aforesaid multi-liquid mixing dispenser with the position control mechanism will be described, taking as an example, the continuous filling method of storing the pre-prepared raw material compositions A and B in the two tank parts, discharging only the raw material composition A in the tip region filling step, discharging only the raw material composition B in the base end region filling step, and appropriately mixing the raw material composition A and the raw material composition B to simultaneously perform the preparation and the filling of each of the "color unit" raw material compositions {the (m-2) types of intermediate region HR raw material compositions} which are the raw materials of the "color unit 2" to the "color unit 4 (= m-2).

In the case where the continuous filling method is executed in the pattern 1, when the raw material composition A and the raw material composition B are mixed in the intermediate region filling step after the tip region filling step is performed by discharging only the raw material composition A, the intermediate region filling step is performed by preparing the intermediate region HR raw material compositions for the respective "color units" with the ratio of the raw material composition A occupying the obtained mixture being reduced in stages, and sequentially discharging these, and thereafter, the base end region filling step is performed by discharging only the raw material composition B. Alternatively, in the case where the pattern 2 is employed, when the raw material composition A and the raw material composition B are mixed in the intermediate region filling step after the base end region filling step is performed by discharging only the raw material composition B, the intermediate region filling step is performed by preparing the intermediate region HR raw material compositions for the respective "color units" with the ratio of the raw material composition B occupying the obtained mixture being reduced in stages, and sequentially discharging them, and thereafter, the tip region filling step is performed by discharging only the raw material composition A.

The following describes a procedure for filling the HR raw material composition in paste form at this time, where, in the expression by a coordinate system whose origin is a given point of the bottom surface in the mold, whose x-axis and y-axis represent the lateral direction and the longitudinal direction of the bottom surface respectively, and whose z-axis represents the height direction, (m-1) pieces of z-coordinates corresponding to the heights of boundaries of m pieces of "color units" that are to be formed in the mold are represented by $z_1$ to $z_{m-1}$ from the bottom surface side. Specifically, the filling is performed by:

(i) setting the position of the discharge port when the filling starts, at the origin;

(ii) moving the position of the discharge port in the x-axis direction and/or the y-axis direction to scan a whole region on the bottom surface once while discharging the HR raw material composition at a predetermined flow rate from the discharge port, to form a mold lowest filling region having a uniform thickness corresponding to the one scanning;

(iii) moving the position of the discharge port in the z-axis direction to a height of an upper surface of the mold lowest filling region or higher after finishing the scanning in (ii);

(iv) moving the position of the discharge port in the x-axis direction and/or the y-axis direction to scan a whole region on the upper surface of the mold lowest filling region as a foundation once while discharging the HR raw material composition at a predetermined flow rate in the same manner as in (ii), to form a grown filling region resulting from addition of a uniform thickness corresponding to the one scanning; and

(v) repeating the operations (iii) and (iv) continuously with the grown filling region as the foundation, and

the varying of the ratio in the raw material composition to be discharged is performed in the operations (ii) to (iv) such that, at an instant when the thickness of the formed grown filling region becomes equal or substantially equal to each of the heights $z_1$ to $z_{m-1}$, the raw material composition for the next "color unit" is discharged from the discharge port.

Consequently, it is possible to efficiently produce a blank having a single layer in a short time. Note that the scanning direction in the filling region may be selected as desired from the x-direction, the y-direction, and a combination of these, and examples thereof include a spiral form starting from the center, a bellows form starting from any of the four corners, and a spiral form starting from any of the four corners. Preferably, only the (height: z-coordinate) of the end point of the scanning is changed with its (x, y) coordinates left as they are and the resultant coordinates are set as the starting point of the next scanning so that the filling is continuously performed. Further, the paste discharge rate during the scanning needs to be appropriately adjusted according to the operation speed of the device, which can be done by deciding an amount that is sufficient and is not excessive in test working in advance. Too large an amount leads to an excessive thickness of the region, and an insufficient amount leads to a size failure of the blank. Therefore, the amount may be appropriately decided in the test working according to the size of a blank to be produced, a required production speed, and so on so as to be neither excessive nor insufficient.

A more detailed description will be given, taking, as an example, the filling step in the case where the section to be cut 1 illustrated in Fig. 1 is produced by the above-described continuous filling method employing the pattern 2, where the color B of the "color unit 6 (= m)" is a color tone corresponding to the color tone of the cervix part of the tooth and its vicinity region, and the color A of the "color unit 1" is a color tone corresponding to the color tone of the tip region of the tooth. In the mold, the "color unit 6 (=m)" 14, the "color unit 5 (= m-1)" 15, the "color unit 4 (= m-2)" 16, the "color unit 3 (= m-3)" 17, the "color unit 2 (= m-4)" 18, and the "color unit 1 (= m-5)" 19 are to be formed from the bottom 2 side. Accordingly, $z_1$ is a height corresponding to the thickness of the "color unit 6", $z_2$ is a height corresponding to the total thickness of the "color unit 6" and the "color unit 5", and similarly, $z_5$ is a height corresponding to the total thickness of the "color unit 6" to the "color unit 2".

Then, in (i) and (ii) above, in a case where the origin is set, for example, at the right front corner of the mold bottom surface 2, while only the raw material composition B which is the raw material of the "color unit 6" is discharged as the HR raw material composition 40 in paste form from the discharge part (discharge port at the nozzle tip), the discharge part is moved, for example, in the arrow direction illustrated in Fig. 3 and the left drawing of Fig. 4, to scan the whole region on the bottom surface 2 once, thereby forming the mold lowest filling region 50 having a uniform thickness corresponding to the one scanning, specifically, having an (average) thickness corresponding to a height equal to the total amount (volume) of the paste discharged in the one scanning divided by the bottom area. Thereafter, in (iii) and (iv), as illustrated in the right drawing in Fig. 4, the discharge part is moved upward from the end point of the one scanning by the aforesaid (average) thickness, and with this point set as the starting point, the whole region on an upper surface of the mold lowest filling region 50 being a foundation (filling region) 51 is scanned once, thereby forming a grown filling region 52 resulting from the addition of a uniform thickness corresponding to the one scanning. Thereafter, from the end point of the scanning, the discharge part is further moved upward by the added thickness, and the whole region of an upper surface on the grown filling region is scanned once, thereby forming a new grown filling region resulting from the addition of a uniform thickness corresponding to the one scanning, and by repeating the above cycle until the thickness (height) of the new grown filling region becomes equal or proximate to $z_1$, the filling for the base end region 11 is performed. As soon as the filling for the base end region 11 ends, a predetermined amount of the raw material composition A is added to the raw material composition B left in the "mixer part", the "mixer part" mixes them to prepare the raw material of the "color unit 5", and thereafter, the discharge part is moved upward by the aforesaid added thickness from the end point of the scanning and performs scanning while discharging this to form a new grown filling region. These operations are repeated until the thickness (height) of the new grown filling region becomes equal or proximate to $z_2$, thereby performing the filling for the "color unit 5". Thereafter, the raw material of the "color unit 4" is prepared by varying the mixture ratio of the raw material composition B and the raw material composition A (increasing the ratio of the raw material composition A), and thereafter the discharge part is moved upward from the end point of the scanning by the aforesaid added thickness to perform the scanning while discharging this, thereby forming a new grown filling region. These operations are repeated until the thickness (height) of the new grown filling region becomes equal or proximate to $z_3$, thereby performing the filling for the "color unit 4". Then, such a pattern is repeated until the thickness (height) of a new grown filling region becomes equal or proximate to $z_{5\,(=m-1)}$, thereby performing the filling for the intermediate region 12. Then, finally, the filling for the "color unit 1", that is, the filling for the tip region 13 is performed by discharging only the raw material composition A, and the filling step is finished.

At a stage when the filling for one blank is finished after starting in the pattern 2, the paste for the tip region is left in the discharge port, and therefore, at the time of the filling for the next blank, the filling is preferably started in the pattern 1 from the tip region. Thus alternately performing the pattern 1 and the pattern 2 enables the continuous production of many blanks.

Note that the filling method using the suitable filling device is not limited to the above-described method, and can be, for example, such that the device illustrated in Fig. 2 has a two-stage structure in which its mixer part 24 and discharge part 25 are separated, and m-2 pieces of (for example, four) "tank parts" and so on are provided between the mixer part 24 and the discharge part 25 downstream thereof, the raw material composition B and the raw material composition A are stored in the two storage parts 21a, 21b on the upstream part (first stage), the mixer part 24 appropriately mixes them to prepare the raw material pastes of the "color unit 2" to the "color unit 4" {the (m-2) types of intermediate region HR raw material compositions}, and they are stored in the downstream (second-stage) four "storage parts", whereby the raw material composition B and the raw material composition A can be supplied as they are to the discharge part 25 through the mixer part 24 and discharged, and the pastes stored in the second-stage "tank parts" each can be discharged independently from the discharge part 25.

2-5. The curing step

The curing step is performed to polymerization-cure the HR raw material composition in paste form filled in the mold to turn it into a workable mill blank. The condition for curing the HR raw material composition can be appropriately selected according to the kind of the used polymerization initiator. For example, polymerization temperature in a case where a thermal polymerization initiator is used is preferably 60 to 200°C, more preferably 70 to 150°C, and still more preferably 80 to 130°C. The polymerization at a temperature of 60°C or lower results in an insufficient polymerization reaction and thus weaker strength of the blank for dental cutting, leading to problems such as the occurrence of a crack and deterioration in the physical properties of a worked object. On the other hand, the polymerization at a temperature of 200°C or higher leads to the progress of the discoloration of the resin component in HR, making it difficult to obtain color tone compatibility with the natural tooth, which is the effect of the present invention. In a case where a photopolymerization initiator is used, it is preferable to perform the light irradiation in parallel with the filling such that the thickness of an object to be cured falls within 5 mm. If the thickness exceeds 5 mm, since light transmission into HR is difficult, long-time continuous irradiation is required or a physical property failure tends to be caused. For the purpose of further improving the physical properties, the thermal polymerization initiator may be co-used. In this case, the thermal polymerization may be performed after the above-described step.

An atmosphere during the curing is not limited, but heating is preferably performed under an atmosphere of inert gas such as nitrogen to prevent a color tone change due to oxidation deterioration. A device used for the heating is not limited as long as it is a device capable of heating the cured body to a desired temperature, but a forced-convection oven is usually more excellent in uniformity of inner temperature than a natural-convection type and thus is preferable. Further, a method to set the inert gas atmosphere is not limited, either, and for example, a hermetic container for storing the cured body may be additionally used, or the hermetic structure may be given to the oven itself to replace the inner atmosphere. In a case where the oven does not have the hermetic structure, it is also possible to use a method of continuously feeding the atmosphere gas.

Further, pressure during the curing may be a normal pressure, but the curing is preferably performed under pressurization to 0.1 MPa to 0.9 MPa and preferably 0.2 MPa to 0.6 MPa. It is possible to perform the thermal polymerization under such pressurization by using a pressure container capable of heating, such as a pressure oven or an autoclave.

Then, post-processes such as heat treatment, grinding, cutting, attachment of a holding tool, and printing can be applied to the obtained hybrid resin as required. Further, as required, a holding pin for fixing the blank for dental cutting to a cutting machine may be bonded. The blank holding pin is not limited as long as it has such a shape as to be capable of fixing the blank to the cutting machine, and it need not be provided depending on the shape of the blank and the requirement by the cutting machine. As the material of the blank holding pin, stainless steel, brass, aluminum, or the like is used. A method for fixing the blank holding pin to the resin material for dental cutting may be a method such as fitting or screwing instead of the aforesaid bonding, and the bonding method is not limited either, and any of various commercially available isocyanate-based, epoxy-based, urethane-based, silicone-based, and acryl-based adhesives is usable.

{Examples}

Hereinafter, for a more specific description of the present invention, Examples and Comparative Examples will be described, but the present invention is by no means limited by these. Note that in Examples and Comparative Examples, a "color unit" is represented by "CU", and a "color unit 1" is represented by "CU1".

{Example 1}

(1) Preparation of a "CU1" raw material composition A (referred to also as a paste A) and a "CU5 (= m)" raw material composition B (referred to also as a paste B) giving HR of a color: B

1,6-bis(methacryl-ethyloxycarbonyl amino)trimethylhexane: 70 parts by mass, triethylene glycol dimethacrylate: 30 parts by mass, and benzoyl peroxide: 1.0 parts by mass which were as a polymerizable monomer composition, and 50 parts by mass of an organic-inorganic composite filler with a 12 $\mu$m average particle diameter (containing 83% by mass of spherical silica zirconia with a 0.2 $\mu$m average particle diameter treated with $\gamma$-methacryloyloxy propyltrimethoxysilane (an organic component is the same as that of a cured body of the polymerizable monomer composition)) and 50 parts by mass of spherical silica zirconia with a 0.2 $\mu$m average particle diameter treated with $\gamma$-methacryloyloxy propyltrimethoxysilane which were as a powder composition were uniformly mixed using a planetary mixer to obtain a base polymerizable and curable composition. Further, a red pigment (pigment red 166), a yellow pigment (pigment yellow 95), a blue pigment (pigment blue 60), and a white pigment (titanium dioxide) were compounded as pigment components to 100 parts by mass of the base polymerizable and curable composition, thereby preparing a "color unit 1" raw material composition A1 (referred to also as a paste A1), and a red pigment (pigment red 166), a yellow pigment (pigment yellow 95), a blue pigment (pigment blue 60), and a white pigment (titanium dioxide) were compounded as pigment components to 100 parts by mass of the same base polymerizable and curable composition, thereby preparing a "color unit m" raw material composition B1 (referred to also as a paste B1). Regarding these compositions A1 and B1, cured body samples with a 1 mm thickness were fabricated, and when their L*, a*, and b* were measured using a spectrophotometer ("TC-1800MKII" manufactured by Tokyo Denshoku) under the measurement conditions of a halogen lamp: 12 V 100 W", a measurement wavelength range 380 to 780 nm, a reflected light 0° d method (JIS Z8722), measurement area 5 mm$\phi$, white background (state where a standard white plate was laid on the cured body to shut off light). As a result, the following results were obtained: L* = 74.0, a* = -3.6, and b* = 8.5 in the sample of the paste A1 (tip region color A1: corresponding to the color tone of an incisal edge with A1 vita shade), and L* = 68.6, a* = -2.5, and b* = 17.0 in the sample of the paste B1 (base end region color B1: corresponding to the color tone of a tooth cervix part side with A3 vita shade), and a color difference: $\Delta E_{AB}$ between the tip region color A1 and the base end region color B1 found from these was 10.1.

(2) Production of HR-based mill blanks

Using a commercially available volumetric-metering two-liquid mixing dispenser (with a position control mechanism) having the same basic structure as that of the device illustrated in Fig. 2 and a mold for filling whose inner bottom surface had a shape with an 18.0 mm $\times$ 14.5 mm and whose height was 14.5 mm, five HR raw material compositions for CUs in paste form were filled in the mold by a continuous filling method.

Specifically, the paste A1 and the paste B1 which were both vacuum-defoamed were stored in one and the other of the two "tank parts" respectively, and the filling for "CU1" (tip region) with a 3.6 mm thickness, "CU2" to "CU4" (intermediate regions) all with a 2.4 mm thickness, and a "CU5" (base end region) with a 3.6 mm thickness was performed in the pattern 1. At this time, mixture ratios A1:B1 of the paste A1 and the paste B1 in the HR raw material compositions for "CU2" to "CU4" were set as follows. Further, L*, a*, and b* were measured using separately prepared 1 mm-thick cured body samples of the aforesaid HR raw material compositions, and the results were as follows.

| HR raw material composition | A1:B1 | L* | a* | b* |
|---|---|---|---|---|
| for "CU2" | 75:25 | 72.7 | -3.3 | 10.6 |
| for "CU3" | 50:50 | 71.3 | -3.1 | 12.8 |
| for "CU4" | 25:75 | 70.0 | -2.8 | 14.9 |

Note that color differences: $\Delta E_{ab}$ (where a represents the number assigned to a lower color unit, b represents the number assigned to an upper color unit) between the colors of two adjacent "CUs" found from the above results are $\Delta E_{12} = 2.5$, $\Delta E_{23} = 2.6$, $\Delta E_{34} = 2.5$, and $\Delta E_{45} = 2.5$.

Next, the filled mold was transferred into a heating/pressure device, the curing was caused by fifteen-hour heating at 95°C in parallel with the pressurization to 0.4 MPa under a nitrogen atmosphere, followed by demolding and deburring, whereby four sections to be cut were fabricated, and further holding pins for CAM were bonded to these, whereby four HR-based mill blanks were produced.

(3) Evaluation of the HR-based mill blanks

Using the obtained HR-based mill blanks, crowns (tooth crowns) for a canine and molars were fabricated with a CAD/CAM system, and their appearances were evaluated. Details of the fabricated prostheses and their fabrication and evaluation methods, and the evaluation results are shown below.

<The fabricated crowns and their fabrication method>

A mandibular model where abutment teeth fabricated for the canine and the molars (first premolar, second premolar, and first molar) using core materials were arranged was scanned with a scanner for models, tooth crown STL data corresponding to the abutment teeth were created using bundled CAD software (manufactured by DENTALWING), and further tool paths were created using CAM software (WORKING DENTAL: manufactured by Vero Software). Using the created paths, the crowns A to D for the canine and the molars were fabricated with a cutting machine (DWX-52D: manufactured by DGSHAPE). Note that the heights of the fabricated various crowns are as follows.

·mandibular canine crown (crown A): height 12 mm
·mandibular first premolar crown (crown B): height 8 mm
·mandibular second premolar crown (crown C): height 5.5 mm
·mandibular first molar crown (crown D): height 4.5 mm

<The evaluation method of the crowns and the evaluation results>

To confirm whether the crowns fabricated from the fabricated HR-based mill blanks reproduced the gradations of the natural teeth irrespective of the crown length (height) to make it possible to obtain natural three-dimensional effects after treatment, the following appearance comparison between the crowns and harmonic property evaluation were conducted.

[The appearance comparison between the crowns]

Using dental resin cement (ESTECEM II universal color: manufactured by Tokuyama Dental), the fabricated crowns A to D were put on the abutment teeth of the mandibular model to which the abutment teeth were fixed, the appearances were compared between the adjacent crowns (different in size), and the evaluation was conducted based on the following evaluation criteria. The evaluation resulted in excellent all between the crowns A, B, between the crowns B, C, and between the crowns C, D.
Evaluation criteria

excellent: They are harmonic with almost no appearance difference.
good: An appearance difference is on a level recognized by careful observation.
passing: An appearance difference is recognized by ordinary observation but is on an allowable level.
failing: An appearance difference is large, and an incompatible sense is felt when they are arranged side by side.

[The harmonic property evaluation]

A commercially available complete set of artificial teeth made of dental prostheses (they are commercially available porcelain teeth that are fabricated using a porcelain system and are used in typical aesthetic restoration. Similarly to dental prostheses in ordinary private pay treatment, they are made to reproduce the structures of teeth by stacking and building and have appearances close to those of natural teeth) in which the color tone of a cervix part side corresponds to the shade of the base end region color B1: VITA-A3 color was prepared, and in a mandibular model to which an abutment tooth corresponding to one of the crowns A to D and dental prostheses other than the aforesaid abutment tooth were fixed, on the abutment tooth, the crown (one conforming to this) was put in the above-described manner, whereby an evaluation sample (the number of the crowns = 4) was fabricated. Separately, a maxillary model in which all the dental prostheses were fixed was fabricated, and color appearance was observed in the state where the mandibular and maxillary models were occluded, and when the harmonic properties were evaluated based on the following evaluation criteria, the crown A was evaluated as excellent, the crown B was evaluated as excellent, the crown C was evaluated as excellent, and the crown D was evaluated as excellent.

excellent: The crown restoration part is harmonic with the surroundings, and a color tone transition of the restoration part is also equivalent to that of the artificial tooth.

good: The crown restoration part is on a recognizable level by careful observation, and the color tone transition of the restoration part is close to that in the artificial tooth.

passing: Though the crown restoration part can be recognized by ordinary observation, it is an allowable level.

failing: The restoration part can be recognized at a glance and gives an incompatible sense.

{Examples 2 to 11}

(1) Preparation of a paste A and a paste B

The following paste A2 to be the paste A and the following pastes B2 to B3 to be the paste B were prepared in the same manner except that the type and amount of compounded pigments were changed.

paste A2 (tip region color A2: corresponding to the color tone of an incisal edge with A3 vita shade)

$$L* = 70.8, a* = -3.3, b* = 12.6$$

paste B2 (base end region color B2: corresponding to the color tone of a cervix part side with A3.5 vita shade)

$$L* = 66.0, a* = -1.5, b* = 21.0$$

paste B3 (base end region color B3: corresponding to the color tone of a cervix part side with A4 vita shade)

$$L* = 62.5, a* = -0.5, b* = 24.5$$

(2) Production of HR-based mill blanks

Combinations each composed of one type selected from the pastes A including the paste A1 and one type selected from the pastes B including the paste B1 were used to fabricate four sections to be cut with the number n of CUs = 5 in the same manner as in Example 1, and four HR-based mill blanks were further produced in the same manner as in Example 1. Hereafter, the combination of the pastes, $\Delta E_{AB}$, mixture ratios A:B of the paste A and the paste B at the time of the preparation of HR raw material compositions for "CU2" to "CU4", L*, a*, and b* using 1 mm samples made of HR raw material composition cured bodies prepared in the same manner, and so on in each Example are shown below.

Example 2: The paste A2 and the paste B1 are used ($\Delta E_{AB} = 5.0$).

| HR raw material composition | A2: B1 | L* | a* | b* |
|---|---|---|---|---|
| for "CU2" | 75:25 | 70.8 | -3.3 | 12.6 |
| for "CU3" | 50:50 | 69.7 | -2.9 | 14.8 |
| for "CU4" | 25:75 | 69.2 | -2.7 | 15.9 |

$$\Delta E_{12} = 1.2, \ \Delta E_{23} = 1.3, \ \Delta E_{34} = 1.2, \ \Delta E_{45} = 1.3$$

Example 3: The paste A1 and the paste B2 are used ($\Delta E_{AB} = 15.0$).

| HR raw material composition | A1: B2 | L* | a* | b* |
|---|---|---|---|---|
| for "CU2" | 75:25 | 72.0 | -3.1 | 11.6 |
| for "CU3" | 50:50 | 70.0 | -2.6 | 14.8 |

(continued)

| HR raw material composition | A1: B2 | L * | a * | b* |
| for "CU4" | 25:75 | 68.0 | -2.0 | 17.9 |

$$\Delta E_{12} = 3.7, \ \Delta E_{23} = 3.8, \ \Delta E_{34} = 3.7, \ \Delta E_{45} = 3.7$$

Example 4: The paste A1 and the paste B3 ($\Delta E_{AB} = 19.9$)

| HR raw material composition | A1: B3 | L* | a* | b* |
| for "CU2" | 75:25 | 71.1 | -2.8 | 12.5 |
| for "CU3" | 50:50 | 68.3 | -2.1 | 16.5 |
| for "CU4" | 25:75 | 65.4 | -1.3 | 20.5 |

$$\Delta E_{12} = 5.0, \ \Delta E_{23} = 4.9, \ \Delta E_{34} = 5.0, \ \Delta E_{45} = 5.0$$

Examples 5 to 7: In Examples 5, 6, and 7, HR-based mill blanks were fabricated with the same combinations of the pastes as those in Examples 1, 2, and 3 respectively in the same manner as in Examples 1 to 4 except that the ratios of the pastes were appropriately adjusted such that color differences $\Delta E_{12}$ to $\Delta E_{45}$ had values shown in Table 1.

Examples 8 to 11

HR-based mill blanks were fabricated in the same manner as in Example 1 except that an incisal edge filling height (corresponding to the thickness of CU1), a base end filling height (corresponding to the thickness of CU5), and a CU filling height in intermediate regions (corresponding to the thickness of CU2 to CU4) were set as shown in Table 1.

{Table 1}

| EXAMPLE No. | NUMBER OF CUs m | FILLING HEIGHT FOR EACH CU (mm) | | | AB COLOR DIFFERENCE | COLOR DIFFERENCE BETWEEN ADJACENT CUs | | | |
| | | TIP | BASE END | INTERMEDIATE | | TIP SIDE | INTERMEDIATE REGION $\Delta Ec$ | | BASE END SIDE |
| | | CU1 | Cu 5 | CU2~CU4 | $\Delta E_{AB}$ | $\Delta E_{12}$ | $\Delta E_{23}$ | $\Delta E_{34}$ | $\Delta E_{45}$ |
| 1 | | | | | 10.1 | 2.5 | 2.6 | 2.5 | 2.5 |
| 2 | | | | | 5.0 | 1.2 | 1.3 | 1.2 | 1.3 |
| 3 | | | | | 15.0 | 3.7 | 3.8 | 3.7 | 3.7 |
| 4 | | 3.6 | 3.6 | 2.4 | 19.9 | 5.0 | 4.9 | 5.0 | 5.0 |
| 5 | | | | | 5.0 | 1.0 | 1.3 | 1.3 | 1.4 |
| 6 | 5 | | | | 10.0 | 2.0 | 2.5 | 2.5 | 3.0 |
| 7 | | | | | 15.0 | 3.0 | 3.5 | 4.0 | 4.5 |
| 8 | | 2.2 | 2.2 | 3.4 | 10.1 | 2.5 | 2.5 | 2.5 | 2.5 |
| 9 | | 2.9 | 2.9 | 2.9 | 10.1 | 2.5 | 2.5 | 2.5 | 2.5 |
| 10 | | 4.4 | 4.4 | 1.9 | 10.1 | 2.5 | 2.5 | 2.5 | 2.5 |
| 11 | | 5.1 | 5.1 | 1.5 | 10.1 | 2.5 | 2.5 | 2.5 | 2.5 |

(3) Evaluation of the HR-based mill blanks

Regarding the obtained HR-based mill blanks, crowns were fabricated as in Example 1, and evaluation was conducted. Table 2 shows the results. Note that the harmonic property evaluation in Examples was conducted using full teeth sets of dental prostheses having the color tone of the cervix part side with the same shade as the shade of the used paste B. For example, in Example 2, since the paste B1 is used, the evaluation is conducted using the same full teeth set of the dental prostheses as that used in Example 1, and in Example 3, the evaluation is conducted using a full teeth set of dental prostheses having the cervix part side color tone of A3.5 which is the shade of the paste B2.

{Table 2}

| EXAMPLE No. | RESULT OF APPEARANCE COMPARISON BETWEEN CROWNS | | | RESULT OF HARMONIC PROPERTY EVALUATION | | | |
|---|---|---|---|---|---|---|---|
| | A-B | B-C | C-D | A | B | C | D |
| 1 | excellent | excellent | excellent | excellent | excellent | excellent | excellent |
| 2 | excellent | excellent | excellent | good | good | passing | passing |
| 3 | excellent | excellent | excellent | excellent | excellent | excellent | excellent |
| 4 | passing | passing | passing | passing | passing | passing | passing |
| 5 | excellent | excellent | excellent | passing | passing | passing | passing |
| 6 | excellent | excellent | excellent | excellent | excellent | excellent | excellent |
| 7 | excellent | excellent | excellent | excellent | excellent | excellent | excellent |
| 8 | passing | passing | passing | good | good | good | good |
| 9 | good | good | good | good | good | good | good |
| 10 | excellent | excellent | excellent | good | good | good | good |
| 11 | excellent | excellent | excellent | passing | passing | passing | passing |

{Examples 12 to 24}

The number m of CUs was set to 7, and in Examples 12 to 20, HR-based mill blanks were fabricated in the same manner as in Examples 1 to 4 without changing the filling heights of the tip region and the base end region and with the filling height of CUs in the intermediate regions being set to 1.5 mm, and in Examples 21 to 24, HR-based mill blanks were fabricated in the same manner as in Example 1 with the filling height of CUs being changed as shown in Table 3. Thereafter, they were worked into crowns in the same manner as in Example 1, and evaluation was conducted. Table 4 shows the evaluation results. Note that, in Example 13, a paste in which a small amount of the paste B1 was mixed with the paste A1 to obtain $\Delta E_{AB} = 8.1$ was used as the paste A.

{Table 3}

| EXAMPLE No. | NUMBER OF CUs m | FILLING HEIGHT FOR EACH CU (mm) | | | AB COLOR DIFFERENCE | COLOR DIFFERENCE BETWEEN ADJACENT CUs | | | | | | |
|---|---|---|---|---|---|---|---|---|---|---|---|---|
| | | TIP | BASE END | INTERMEDIATE | | TIP SIDE | INTERMEDIATE REGION ΔEc | | | | | BASE END SIDE |
| | | CU1 | Cu7 | CU2~CU6 | $\Delta E_{AB}$ | $\Delta E_{12}$ | $\Delta E_{23}$ | $\Delta E_{34}$ | $\Delta E_{45}$ | $\Delta E_{56}$ | | $\Delta E_{67}$ |
| 12 | | | | | 10.1 | 1.7 | 1.7 | 1.7 | 1.7 | 1.7 | | 1.7 |
| 13 | | | | | 8.1 | 1.4 | 1.4 | 1.4 | 1.4 | 1.4 | | 1.4 |
| 14 | | | | | 15.0 | 2.5 | 2.5 | 2.5 | 2.5 | 2.5 | | 2.5 |
| 15 | | | | | 19.9 | 3.3 | 3.3 | 3.3 | 3.3 | 3.3 | | 3.3 |
| 16 | | 3.6 | 3.6 | 1.5 | 10.0 | 1.3 | 1.3 | 1.5 | 1.5 | 2.0 | | 2.4 |
| 17 | | | | | 10.0 | 2.4 | 2.0 | 1.5 | 1.5 | 1.3 | | 1.3 |
| 18 | 7 | | | | 10.0 | 1.3 | 1.5 | 2.2 | 2.2 | 1.5 | | 1.3 |
| 19 | | | | | 15.0 | 1.5 | 2.0 | 2.5 | 2.5 | 3.0 | | 3.5 |
| 20 | | | | | 20.0 | 2.5 | 3.0 | 3.0 | 3.5 | 4.0 | | 4.0 |
| 21 | | 2.2 | 2.2 | 2.0 | 10.1 | 1.7 | 1.7 | 1.7 | 1.7 | 1.7 | | 1.7 |
| 22 | | 2.9 | 2.9 | 1.7 | 10.1 | 1.7 | 1.7 | 1.7 | 1.7 | 1.7 | | 1.7 |
| 23 | | 4.4 | 4.4 | 1.2 | 10.1 | 1.7 | 1.7 | 1.7 | 1.7 | 1.7 | | 1.7 |
| 24 | | 5.1 | 5.1 | 0.9 | 10.1 | 1.7 | 1.7 | 1.7 | 1.7 | 1.7 | | 1.7 |

{Table 4}

| EXAMPLE No. | RESULT OF APPEARANCE COMPARISON BETWEEN CROWNS | | | RESULT OF HARMONIC PROPERTY EVALUATION | | | |
|---|---|---|---|---|---|---|---|
| | A-B | B-C | C-D | A | B | C | D |
| 12 | excellent | excellent | excellent | excellent | excellent | excellent | excellent |
| 13 | excellent | excellent | excellent | good | good | passing | passing |
| 14 | excellent | excellent | excellent | excellent | excellent | excellent | excellent |
| 15 | passing | passing | passing | passing | passing | good | good |
| 16 | excellent | excellent | excellent | excellent | excellent | excellent | excellent |
| 17 | excellent | excellent | excellent | excellent | excellent | excellent | excellent |
| 18 | excellent | excellent | excellent | excellent | excellent | excellent | excellent |
| 19 | excellent | excellent | excellent | excellent | excellent | excellent | excellent |
| 20 | passing | passing | passing | good | good | good | good |
| 21 | passing | passing | passing | good | good | good | good |
| 22 | good | good | good | excellent | excellent | excellent | excellent |
| 23 | excellent | excellent | excellent | excellent | excellent | excellent | excellent |
| 24 | excellent | excellent | excellent | passing | passing | passing | passing |

{Examples 25 to 35}

The number m of CUs was set to 9, and in Examples 25 to 31, HR-based mill blanks were fabricated in the same manner as in Examples 1 to 4 without changing the filling heights of the tip region and the base end region and with the filling height of CUs in the intermediate regions set to 1.5 mm, and in Examples 32 to 35, HR-based mill blanks were fabricated in the same manner as in Example 1 with the filling height for CUs being changed as shown in Table 5. Thereafter, they were worked into crowns in the same manner as in Example 1, and evaluation was conducted. Table 6 shows the evaluation results. Note that, in Example 26, a paste in which a small amount of the paste B1 was mixed with the paste A1 to obtain

$\Delta E_{AB}$ = 8.1 was used as the paste A.

{Table 5}

| EXAMPLE No. | NUMBER OF CUs m | FILLING HEIGHT FOR EACH CU (mm) | | | AB COLOR DIFFERENCE | COLOR DIFFERENCE BETWEEN ADJACENT CUs | | | | | | | | |
|---|---|---|---|---|---|---|---|---|---|---|---|---|---|---|
| | | TIP | BASE END | INTERMEDIATE | | TIP SIDE | INTERMEDIATE REGION $\Delta Ec$ | | | | | | | BASE END SIDE |
| | | CU1 | Cu 9 | CU2~CU 8 | $\Delta E_{AB}$ | $\Delta E_{12}$ | $\Delta E_{23}$ | $\Delta E_{34}$ | $\Delta E_{45}$ | $\Delta E_{56}$ | $\Delta E_{67}$ | $\Delta E_{78}$ | $\Delta E_{89}$ |
| 25 | | | | | 10.1 | 1.3 | 1.3 | 1.3 | 1.3 | 1.3 | 1.3 | 1.3 | 1.3 |
| 26 | | | | | 8.1 | 1.0 | 1.0 | 1.0 | 1.0 | 1.0 | 1.0 | 1.0 | 1.0 |
| 27 | | | | | 15.0 | 1.9 | 1.9 | 1.9 | 1.9 | 1.9 | 1.9 | 1.9 | 1.9 |
| 28 | | 3.6 | 3.6 | 1.0 | 19.9 | 2.5 | 2.5 | 2.5 | 2.5 | 2.5 | 2.5 | 2.5 | 2.5 |
| 29 | | | | | 10.0 | 1.0 | 1.0 | 1.3 | 1.3 | 1.3 | 1.3 | 1.3 | 1.5 |
| 30 | 9 | | | | 15.0 | 1.3 | 1.3 | 1.5 | 1.5 | 2.0 | 2.0 | 2.5 | 2.9 |
| 31 | | | | | 20.0 | 1.5 | 2.0 | 2.0 | 2.5 | 2.5 | 3.0 | 3.0 | 3.5 |
| 32 | | 2.2 | 2.2 | 1.5 | 10.1 | 1.3 | 1.3 | 1.3 | 1.3 | 1.3 | 1.3 | 1.3 | 1.3 |
| 33 | | 2.9 | 2.9 | 1.2 | 10.1 | 1.3 | 1.3 | 1.3 | 1.3 | 1.3 | 1.3 | 1.3 | 1.3 |
| 34 | | 4.4 | 4.4 | 0.8 | 10.1 | 1.3 | 1.3 | 1.3 | 1.3 | 1.3 | 1.3 | 1.3 | 1.3 |
| 35 | | 5.1 | 5.1 | 0.6 | 10.1 | 1.3 | 1.3 | 1.3 | 1.3 | 1.3 | 1.3 | 1.3 | 1.3 |

{Table 6}

| EXAMPLE No. | RESULT OF APPEARANCE COMPARISON BETWEEN CROWNS | | | RESULT OF HARMONIC PROPERTY EVALUATION | | | |
|---|---|---|---|---|---|---|---|
| | A-B | B-C | C-D | A | B | C | D |
| 25 | excellent | excellent | excellent | excellent | excellent | excellent | excellent |
| 26 | excellent | excellent | excellent | passing | passing | passing | passing |
| 27 | excellent | excellent | excellent | excellent | excellent | excellent | excellent |
| 28 | good | good | good | good | good | good | good |
| 29 | excellent | excellent | excellent | passing | passing | passing | passing |
| 30 | excellent | excellent | excellent | excellent | excellent | excellent | excellent |
| 31 | passing | good | good | good | good | good | good |
| 32 | passing | passing | passing | good | good | good | good |
| 33 | good | good | good | excellent | excellent | good | good |
| 34 | excellent | excellent | excellent | excellent | excellent | excellent | excellent |
| 35 | excellent | excellent | excellent | passing | passing | passing | passing |

{Comparative Example 1}

A paste B4 giving the base end region color B where L* = 69.0, a* = -2.8, and b* = 16.0 which correspond to a color tone having undergone fine adjustment with reference to a vita shade A3 cervix part such that it became slightly paler was prepared in the same manner as in the preparation of the paste B1 except that the types and amounts of compounded pigments were changed, and the paste B4 was used as the paste B and the aforesaid paste A1 was used as the paste A ($\Delta E_{AB}$ was 3.9), and a HR-based mill blank was fabricated in the same manner as in Example 1. At this time, a mixture ratio of the pastes A1 and B4 was appropriately adjusted such that color differences $\Delta E$ between adjacent CUs became the values shown in Table 7, whereby HR raw material compositions for intermediate regions (CU2 to CU4) were prepared.

## {Table 7}

| COMPARATIVE EXAMPLE No. | NUMBER OF CUs m | FILLING HEIGHT FOR EACH CU (mm) | | | AB COLOR DIFFERENCE | COLOR DIFFERENCE BETWEEN ADJACENT CUs $\Delta E$ | | | |
|---|---|---|---|---|---|---|---|---|---|
| | | TIP | BASE END | INTERMEDIATE | | | | | |
| | | CU1 | Cum | CU2~CU(m-1) | $\Delta E_{AB}$ | TIP SIDE | INTERMEDIATE REGION $\Delta Ec$ | | BASE END SIDE |
| 1 | 5 | 3.6 | 3.6 | 2.4 | 3.9 | $\Delta E_{12}: 1.0$ | $\Delta E_{23}: 0.9$ | $\Delta E_{34}: 1.0$ | $\Delta E_{45}: 1.0$ |
| 2 | | 3.6 | 3.6 | 1.5 | 10 | $\Delta E_{12}: 3.0$ | $\Delta E_{23}: 0.5$ | $\Delta E_{34}: 3.5$ | $\Delta E_{45}: 3.0$ |
| 3 | 3 | 3.6 | 3.6 | 7.3 | 10 | ALL 5.0 | | | |
| 4 | 4 | 3.6 | 3.6 | 3.7 | 10 | ALL 3.3 | | | |
| 5 | 10 | 3.6 | 3.6 | 0.9 | 10 | ALL 1.1 | | | |

The obtained HR-based mill blank was worked into a crown as in Example 1, and evaluation was conducted. Table 8 shows the evaluation results.

## {Table 8}

| COMPARATIVE EXAMPLE No. | APPEARANCE COMPARISON BETWEEN CROWNS | | | HARMONIC PROPERTY EVALUATION | | | |
|---|---|---|---|---|---|---|---|
| | A-B | B-C | C-D | A | B | C | D |
| 1 | good | good | good | failing | failing | failing | failing |
| 2 | good | good | good | failing | failing | failing | failing |
| 3 | failing | failing | failing | failing | failing | failing | failing |
| 4 | failing | failing | failing | good | good | good | good |
| 5 | good | good | good | passing | passing | failing | failing |

{Comparative Example 2}

A HR-based mill blank and a crown were fabricated in the same manner as in Example 1 except that HR raw material compositions for intermediate regions (CU2 to CU4) in which mixture ratios of the pastes A1 and B1 were appropriately adjusted such that color differences ΔE between adjacent CUs became the values shown in Table 7 were prepared to be used, and evaluation was conducted. Table 8 shows the evaluation results.

{Comparative Examples 3 to 5}

HR-based mill blanks and crowns were fabricated in the same manner as in Example 1 such that the number m of CUs, the filling heights for CUs in intermediate regions, and color differences ΔE between the adjacent CUs became the values shown in Table 7, and evaluation was conducted. Table 8 shows the evaluation results.

{Reference Signs List}

1   section to be cut
2   bottom surface
10   gradation structure of present invention
11   base end region (base end region color B)
12   intermediate region (intermediate region color C: $C_2$ to $C_5$)
13   tip region (tip region color A)

| 14 | color unit 6 (color unit m) |
|---|---|
| 15 | color unit 5 (color unit m-1) |
| 16 | color unit 4 |
| 17 | color unit 3 |
| 18 | color unit 2 |
| 19 | color unit 1 |
| 20 | two-liquid mixing dispenser (multi-liquid mixing dispenser with position control mechanism) |
| 21, 21b | tank part |
| 22a, 22b | pressurizing/pressure-reducing part |
| 23a, 23b | pipe part |
| 24 | mixer part |
| 25 | discharge part |
| 26 | discharge port |
| 27 | arm part |
| 28 | stage part |
| 30 | mold |
| 40 | HR raw material composition in paste form |
| 50 | mold lowest filling region |
| 51 | foundation (filling region) |
| 52 | grown filling region |

**Claims**

1. A mill blank for dental cutting comprising a section to be cut (1) that has a shape of a columnar body whose bottom surface (2) and upper surface are in a substantially identical shape and that is formed of a hybrid resin, the section to be cut having a gradation structure (10) in which color appearance changes in stages in a height direction, where the height direction is a direction from the bottom surface of the columnar body being a base end (11) toward the upper surface which is a tip (13),

   wherein the columnar body is formed of a hybrid resin that is a composite material in which an inorganic filling material is dispersed in a resin matrix,
   wherein, in the gradation structure, a region having one color appearance forms each of "color units" into which the columnar body is divided in the height direction along a plane horizontal to the bottom surface and that each have a predetermined thickness, and five to nine "color units" (14,15,16,17,18,19) different in the color appearance are continuously arranged in the height direction, and
   wherein, when the five to nine "color units" are each assigned a unit number: n (where n is a natural number of 1 to m) out of 1 to m (where m is a natural number representing the number of the units and is within a range of 5 to 9) to be denoted by a "color unit n" such that a value of the number increases in order from the tip side toward the base end side, and a color of the "color unit 1" located at the tip is represented by a "tip region color: A" and a color of the "color unit m" located at the base end is represented by a "base end region color: B",
   a color difference: $\Delta E_{AB}$ between the tip region color: A and the base end region color: B is 5 to 20,
   colors of the "color unit 2" to the "color unit m-1" are all intermediate colors: C between the colors A and B and change from an intermediate color close to A to an intermediate color close to B in stages from the "color unit 2" toward the "color unit m-1",
   a color difference: $\Delta E_C$ between the colors of the adjacent units is 1 to 7, wherein
   the color difference ($\Delta E$) is an index representing a degree of difference between two colors and is defined by the equation (1), which is expressed by using numerical values (L* values, a* values, and b* values) in the coordinate axes in the L*a*b* color space,

$$\text{Equation (1) } \Delta E = \{(\Delta L^*)^2 + (\Delta a^*)^2 + (\Delta b^*)^2\}^{1/2}$$

   wherein in the equation (1), $\Delta L^*$ is represented by the difference between lightness L* of a first color and lightness L* of a second color, $\Delta a^*$ is represented by the difference between chromaticity a* of the first color and chromaticity a* of the second color, and $\Delta b^*$ is represented by the difference between chromaticity b* of the first color and chromaticity b* of the second color,

   **characterized in that** the columnar body is formed of a single layer not having a laminate interface.

2. The mill blank for dental cutting according to claim 1, wherein, with the thickness in the height direction of each of the "color units" being defined as a height of each of the "color units", the heights of the "color unit 1" (19) and the "color unit m" (14) are each 15 to 35% of a whole height of the columnar body, and the height of the "color unit 1" and the height of the "color unit m" are optionally different.

3. A method for producing the mill blank for dental cutting according to claim 1 or 2, the method comprising:

a raw material preparation step of preparing a hybrid resin raw material composition (40) in paste form containing a polymerizable monomer, an inorganic filler, a thermal polymerization initiator, and a coloring agent;
a filling step of filling the hybrid resin raw material composition in a mold (30) corresponding to the shape of the section to be cut (1); and
a curing step of curing the hybrid resin raw material composition filled in the mold by thermal polymerization, wherein the raw material preparation step prepares, as the hybrid resin raw material composition, a "color unit 1" (19) raw material composition A giving a hybrid resin of the color: A and a "color unit m" (16) raw material composition B giving a hybrid resin of the color: B, and by using tanks capable of separately holding the "color unit 1" raw material composition A and the "color unit m" raw material composition B and a discharge device capable of uniformly mixing, at a desired ratio, the two types of hybrid resin raw material compositions held in the tanks and discharging a mixture from a discharge port, mixes the "color unit 1" raw material composition A and the "color unit m" raw material composition B with a mixture ratio of A and B being varied, to prepare the (m-2) types of intermediate region hybrid resin raw material compositions giving the colors: of the "color unit 2" to the "color unit (m-1),
wherein the filling step includes:

a tip region filling step of extruding the "color unit 1" raw material composition A from the discharge port (26) to fill, in the mold, a necessary amount of the "color unit 1" raw material composition A in order to form the "color unit 1";
a base end region filling step of extruding the "color unit m" raw material composition B from the discharge port to fill, in the mold, a necessary amount of the "color unit m" raw material composition B in order to form the "color unit m"; and
an intermediate region filling step of extruding the intermediate region hybrid resin raw material compositions prepared using the discharge device from the discharge port to fill, in the mold, necessary amounts of the intermediate region hybrid resin raw material compositions in order to form the "color unit 2" (18) to the "color unit m-1" (15),
wherein the filling step is performed in either pattern out of:

1) a pattern 1 in which the tip region filling step, the intermediate region filling step, and the base end region filling step are performed in the order mentioned; and
2) a pattern 2 in which the base end region filling step, the intermediate region filling step, and the tip region filling step are performed in the order mentioned, and

wherein, in the pattern 1 and the pattern2, the filling steps are performed by continuously extruding the hybrid resin raw material composition while varying, in stages, a constitution of the hybrid resin raw material composition that is to be extruded from the discharge port.

4. The method for producing the mill blank for dental cutting according to claim 3,

wherein, when a position in the mold is expressed by a coordinate system whose origin is a given point on a bottom surface in the mold and whose x-axis and y-axis represent a lateral direction and a longitudinal direction of the bottom surface respectively and whose z-axis represents a height direction, and (m-1) pieces of z-coordinates corresponding to heights of boundaries between m pieces of the "color units" that are to be formed in the mold are denoted by $z_1$ to $z_{m-1}$ from the bottom surface side,
the filling of each of the types of "color unit" raw material compositions in the mold in the filling step is performed using the discharge device capable of freely controlling a position of the discharge port (26) in a front-rear direction, a left-right direction, and the height direction, by:

(i) setting the position of the discharge port when the filling starts, at the origin;
(ii) moving the position of the discharge port in the x-axis direction and/or the y-axis direction to scan a whole region on the bottom surface once while discharging the hybrid resin raw material composition at a predetermined flow rate from the discharge port, to form a mold lowest filling region having a uniform thickness corresponding to the one scanning;

(iii) moving the position of the discharge port in the z-axis direction to a height of an upper surface of the mold lowest filling region or higher after finishing the scanning in (ii);

(iv) moving the position of the discharge port in the x-axis direction and/or the y-axis direction to scan a whole region on the upper surface of the mold lowest filling region as a foundation once while discharging the hybrid resin raw material composition at a predetermined flow rate in the same manner as in the (ii), to form a grown filling region resulting from addition of a uniform thickness corresponding to the one scanning; and

(v) repeating the operations (iii) and (iv) continuously with the grown filling region as the foundation, and

wherein the varying of the ratio in the hybrid resin raw material composition to be discharged is performed in the operations (ii) to (iv) such that, at an instant when the thickness of the formed grown filling region becomes equal or substantially equal to each of the heights $z_1$ to $z_{m-1}$, the raw material composition for the next "color unit" is discharged from the discharge port.

5. The method for producing the mill blank for dental cutting according to claim 3 or 4,
   wherein the filling step alternately performs the filling of the hybrid resin raw material composition into the mold in the pattern 1 and the filling of the hybrid resin raw material composition into the mold in the pattern 2

**Patentansprüche**

1. Fräsrohling für ein Dentalfräsen, der einen zu fräsenden Abschnitt (1) umfasst, der die Form eines säulenförmigen Körpers aufweist, dessen untere Oberfläche (2) und obere Oberfläche eine im Wesentlichen identische Form aufweisen und der aus einem Hybridharz geformt ist,

   wobei der zu fräsende Abschnitt eine Abstufungsstruktur (10), bei der sich das Farberscheinungsbild stufenweise in Höhenrichtung ändert, aufweist, wobei die Höhenrichtung eine Richtung ist, die von der unteren Oberfläche des säulenförmigen Körpers, welche ein Basisende **(11)** ist, hin zu der oberen Oberfläche, welche eine Spitze (13) ist, verläuft,
   wobei der säulenförmige Körper aus einem Hybridharz geformt ist, bei dem es sich um ein Verbundmaterial, bei dem ein anorganisches Füllmaterial in einer Harzmatrix dispergiert ist, handelt,
   wobei in der Abstufungsstruktur ein Bereich, der ein einzelnes Farberscheinungsbild aufweist, jeweils eine der "Farbeinheiten" bildet, in die der säulenförmige Körper in Höhenrichtung entlang einer zu der unteren Oberfläche horizontal verlaufenden Ebene unterteilt ist und die jeweils eine vorgegebene Dicke aufweisen, und wobei fünf bis neun "Farbeinheiten" (14, 15, 16, 17, 18, 19), die hinsichtlich des Farberscheinungsbilds unterschiedlich sind, in Höhenrichtung fortlaufend angeordnet sind,
   und wobei,
   wenn den fünf bis neun "Farbeinheiten" jeweils eine Einheit-Nummer: n (wobei n eine natürliche Zahl von 1 bis m ist) aus 1 bis m (wobei m eine natürliche Zahl ist, die die Anzahl der Einheiten repräsentiert und in einem Bereich von 5 bis 9 liegt) zugewiesen wird, so dass sie durch eine "Farbeinheit n" in einer solchen Weise bezeichnet werden, dass der Wert der Nummer der Reihe nach von der Seite der Spitze zur Seite des Basisendes zunimmt, und eine Farbe der "Farbeinheit 1", die sich an der Spitze befindet, durch eine "Spitzenbereichsfarbe: A" repräsentiert ist, und eine Farbe der "Farbeinheit m", die sich an dem Basisende befindet, durch eine "Basisendbereichsfarbe: B" repräsentiert ist,
   der Farbunterschied: $\Delta E_{AB}$ zwischen der Spitzenbereichsfarbe: A und der Basisendbereichsfarbe: B 5 bis 20 beträgt,
   die Farben der "Farbeinheit 2" bis zur "Farbeinheit m-1" allesamt Zwischenfarben: C zwischen den Farben A und B sind und sich von einer Zwischenfarbe nahe bei A zu einer Zwischenfarbe nahe bei B stufenweise von der "Farbeinheit 2" hin zu der "Farbeinheit m-1" ändern,
   ein Farbunterschied: $\Delta E_C$ zwischen den Farben der benachbarten Einheiten 1 bis 7 beträgt,
   wobei
   der Farbunterschied ($\Delta E$) ein Index ist, der den Grad des Unterschieds zwischen zwei Farben repräsentiert und der durch die Formel (1) definiert ist, welche durch Verwenden von numerischen Werten (L*-Werte, a*-Werte und b*-Werte) in den Koordinatenachsen in dem L*a*b*-Farbraum ausgedrückt wird,

$$\text{Gleichung (1)} \quad \Delta E = \{(\Delta L^*)^2 + (\Delta a^*)^2 + (\Delta b^*)^2\}^{1/2},$$

wobei in Gleichung (1) $\Delta L^*$ durch den Unterschied zwischen der Helligkeit $L^*$ einer ersten Farbe und der Helligkeit $L^*$ einer zweiten Farbe repräsentiert wird, $\Delta a^*$ durch den Unterschied zwischen der Chromatizität $a^*$ der ersten Farbe und der Chromatizität $a^*$ der zweiten Farbe repräsentiert wird, und $\Delta b^*$ durch den Unterschied zwischen der Chromatizität $b^*$ der ersten Farbe und der Chromatizität $b^*$ der zweiten Farbe repräsentiert wird, **dadurch gekennzeichnet, dass** der säulenförmige Körper von einer einzigen Schicht ohne Laminatgrenzfläche gebildet wird.

2. Fräsrohling für dentales Fräsen gemäß Anspruch 1, wobei, wenn die Dicke in Höhenrichtung jeder der "Farbeinheiten" als Höhe einer jeden der "Farbeinheiten" definiert ist, die Höhen der "Farbeinheit 1" (19) und der "Farbeinheit m" (14) jeweils 15 bis 35 % der Gesamthöhe des säulenförmigen Körpers betragen, und die Höhe der "Farbeinheit 1" und die Höhe der "Farbeinheit m" optional verschieden sind.

3. Verfahren zur Herstellung des Fräsrohlings für dentales Fräsen gemäß Anspruch 1 oder Anspruch 2, wobei das Verfahren Folgendes umfasst:

einen Rohmaterial-Zubereitungsschritt, bei dem eine Hybridharz-Rohmaterialzusammensetzung (40) in Form einer Paste hergestellt wird, die ein polymerisierbares Monomer, einen anorganischen Füllstoff, einen thermischen Polymerisationsstarter und ein Farbmittel enthält,
einen Füllschritt, bei dem die Hybridharz-Rohmaterialzusammensetzung in eine Form (30) gefüllt wird, die der Form des zu fräsenden Abschnitts (1) entspricht, und
einen Aushärtungsschritt, bei dem die in die Form gefüllte Hybridharz-Rohmaterialzusammensetzung durch thermische Polymerisation ausgehärtet wird,
wobei der Rohmaterial-Zubereitungsschritt als Hybridharz-Rohmaterialzusammensetzung eine Rohmaterialzusammensetzung der "Farbeinheit 1" A (19), die ein Hybridharz der Farbe A liefert, und eine Rohmaterialzusammensetzung der "Farbeinheit m" B (16), die ein Hybridharz der Farbe B liefert, herstellt, und unter Verwendung von Tanks, die in der Lage sind, die Rohmaterialzusammensetzung der "Farbeinheit 1" A und die Rohmaterialzusammensetzung der "Farbeinheit m" B getrennt aufzunehmen, und einer Ausgabevorrichtung, die in der Lage ist, die zwei Arten von Hybridharz-Rohmaterialzusammensetzungen, die in den Tanks aufgenommen sind, im gewünschten Verhältnis gleichmäßig zu mischen, das Gemisch über eine Austragsöffnung auszutragen, die Rohmaterialzusammensetzung der "Farbeinheit 1" A und die Rohmaterialzusammensetzung der "Farbeinheit m" B in einem Mischungsverhältnis A zu B, das variiert werden kann, mischt, um die (m-2) Arten von Hybridharz-Rohmaterialzusammensetzungen des Zwischenbereichs, die die Farben: der "Farbeinheit 2" bis zu der "(Farbeinheit m-1)" zu liefern, herzustellen,
wobei der Füllschritt Folgendes umfasst:

einen Füllschritt des Spitzenbereichs, bei dem die Rohmaterialzusammensetzung der "Farbeinheit 1" A aus der Austragsöffnung (26) extrudiert wird, um eine erforderliche Menge der Rohmaterialzusammensetzung der "Farbeinheit 1" A in die Form zu füllen, um die "Farbeinheit 1" zu bilden,
einen Füllschritt des Basisendbereichs, bei dem die Rohmaterialzusammensetzung der "Farbeinheit m" B aus der Austragsöffnung extrudiert wird, um eine erforderliche Menge der Rohmaterialzusammensetzung der "Farbeinheit m" B in die Form zu füllen, um die "Farbeinheit m" zu bilden, und
einen Füllschritt des Zwischenbereichs des Extrudierens der Hybridharz-Rohmaterialzusammensetzungen des Zwischenbereichs, die unter Verwendung der Austragsvorrichtung hergestellt werden, aus der Austragsöffnung, um erforderliche Mengen der Hybridharz-Rohmaterialzusammensetzungen des Zwischenbereichs in die Form zu füllen, um die "Farbeinheit 2" (18) bis zur "Farbeinheit m-1" (15) zu bilden,
wobei der Füllschritt in einem der folgenden Muster durchgeführt wird:

1) einem Muster 1, bei dem der Füllschritt des Spitzenbereichs, der Füllschritt des Zwischenbereichs und der Füllschritt des Basisendbereichs in der genannten Reihenfolge durchgeführt werden; und
2) einem Muster 2, bei dem der Füllschritt des Basisendbereichs, der Füllschritt des Zwischenbereichs und der Füllschritt des Spitzenbereichs in der genannten Reihenfolge durchgeführt werden, und

wobei, bei dem Muster 1 und dem Muster 2, die Füllschritte durchgeführt werden, indem die Hybridharz-Rohmaterialzusammensetzung kontinuierlich extrudiert wird, während gleichzeitig eine Zusammensetzung der Hybridharz-Rohmaterialzusammensetzung, die aus der Austragsöffnung extrudiert werden soll, stufenweise geändert wird.

4. Verfahren zur Herstellung des Fräsrohlings für dentales Fräsen gemäß Anspruch 3, wobei,

wenn eine Position in der Form durch ein Koordinatensystem ausgedrückt wird, dessen Ursprung ein vorgegebener Punkt auf der unteren Oberfläche in der Form ist, und dessen x-Achse und y-Achse eine seitliche Richtung bzw. eine Längsrichtung der unteren Oberfläche repräsentieren, und dessen z-Achse eine Höhenrichtung repräsentiert, und wobei (m-1) Abschnitte von z-Koordinaten, die den Höhen der Grenzen zwischen m Abschnitten der "Farbeinheiten", die in der Form zu bilden sind, entsprechen, von der Seite der unteren Oberfläche her mit $z_1$ bis $z_{m-1}$ bezeichnet werden,

das Füllen der verschiedenen Arten von Rohmaterialzusammensetzungen der "Farbeinheit" in die Form im Füllschritt unter Verwendung der Austragsvorrichtung, die in der Lage ist, die Position der Austragsöffnung (26) in einer Vorwärts-Rückwärts-Richtung, einer Links-Rechts-Richtung und einer Höhenrichtung frei zu steuern, wie folgt durchgeführt wird:

(i) Setzen der Position der Austragsöffnung, wenn das Befüllen beginnt, auf den Ursprung,
(ii) Bewegen der Position der Austragsöffnung in Richtung der x-Achse und/oder in Richtung der y-Achse, um einen gesamten Bereich auf der unteren Oberfläche einmal abzutasten, während die Hybridharz-Rohmaterialzusammensetzung mit einer vorgegebenen Durchflussrate aus der Austragsöffnung ausgetragen wird, um einen Bereich mit niedrigster Befüllung der Form auszubilden, der eine gleichmäßige Dicke entsprechend dem einen Abtastvorgang aufweist,
(iii) Bewegen der Position der Austragsöffnung Richtung der z-Achse auf eine Höhe der oberen Oberfläche des niedrigsten Füllbereichs der Form oder höher, nachdem der Scanvorgang in (ii) abgeschlossen ist,
(iv) Bewegen der Position der Austragsöffnung in Richtung der x-Achse und/oder Richtung der y-Achse, um einen gesamten Bereich auf der oberen Oberfläche des niedrigsten Füllbereichs der Form als Grundlage einmalig abzutasten, während die Hybridharz-Rohmaterialzusammensetzung mit einer vorgegebenen Durchflussrate in der gleichen Weise wie in (ii) ausgetragen wird, um einen gewachsenen Füllbereich auszubilden, der von dem Hinzufügen einer gleichmäßigen Dicke entsprechend dem einen Abtastvorgang herrührt, und
(v) kontinuierliches Wiederholen der Arbeitsgänge (iii) und (iv) mit dem gewachsenen Füllbereich als Grundlage, und

wobei das Verändern des Verhältnisses der Hybridharz-Rohmaterialzusammensetzung, die ausgetragen werden soll, in den Arbeitsgängen (ii) bis (iv) in einer solchen Weise erfolgt, dass zu einem Zeitpunkt, an dem die Dicke des gebildeten gewachsenen Füllbereichs gleich oder im Wesentlichen gleich jeder der Höhen $z^1$ bis $z^{m-1}$ ist, die Rohmaterialzusammensetzung für die nächste "Farbeinheit" aus der Austragsöffnung ausgetragen wird.

**5.** Verfahren zur Herstellung des Fräsrohlings für dentales Fräsen gemäß Anspruch 3 oder Anspruch 4, wobei in dem Füllschritt abwechselnd das Füllen der Hybridharz-Rohmaterialzusammensetzung in die Form im Muster 1 und das Füllen der Hybridharz-Rohmaterialzusammensetzung in die Form im Muster 2 durchgeführt wird.

**Revendications**

**1.** Ébauche de fraise pour découpe dentaire comprenant une section à découper (1) qui a la forme d'un corps en colonne dont la surface inférieure (2) et la surface supérieure ont une forme sensiblement identique et qui est formée d'une résine hybride, la section à découper ayant une structure de gradation (10) dans laquelle l'apparence de couleur change par étapes dans le sens de la hauteur, le sens de la hauteur étant une direction allant de la surface inférieure du corps en colonne qui est une extrémité de base (11) vers la surface supérieure qui est une pointe (13),

le corps en colonne étant formé d'une résine hybride qui est un matériau composite dans lequel un matériau de remplissage inorganique est dispersé dans une matrice de résine,
dans la structure de gradation, une zone ayant un aspect de couleur formant chacune des « unités de couleur » en lesquelles le corps en colonne est divisé dans le sens de la hauteur le long d'un plan horizontal à la surface inférieure et qui ont chacune une épaisseur prédéterminée, et cinq à neuf "unités de couleur" (14, 15, 16, 17, 18, 19) différentes dans l'apparence de couleur étant disposées en continu dans le sens de la hauteur, et
lorsque les cinq à neuf "unités de couleur" sont chacune affectées d'un numéro d'unité : n (où n est un nombre naturel de 1 à m) sur 1 à m (où m est un nombre naturel représentant le nombre d'unités et est dans une plage de 5 à 9) à désigner par une "unité de couleur n" de sorte qu'une valeur du numéro augmente dans l'ordre du côté pointe vers le côté extrémité de base, et qu'une couleur de l'« unité de couleur 1 » située au niveau de la pointe soit représentée par une « couleur de la zone de pointe : A » et une couleur de l'« unité de couleur m » située à l'extrémité de base soit représentée par une « couleur de la zone d'extrémité de base : B »,

une différence de couleur : $\Delta E_{AB}$ entre la couleur de la zone de pointe : A et la couleur de la zone d'extrémité de base : B étant de 5 et à 20,

les couleurs de « l'unité de couleur 2 » à « l'unité de couleur m-1 » étant toutes des couleurs intermédiaires : C entre les couleurs A et B et passant d'une couleur intermédiaire proche de A à une couleur intermédiaire proche de B par étapes de « l'unité de couleur 2 » à « l'unité de couleur m-1 »,

une différence de couleur : $\Delta E_C$ entre les couleurs des unités adjacentes étant de 1 à 7,

la différence de couleur ($\Delta E$) étant un indice représentant un degré de différence entre deux couleurs et étant définie par l'équation (1), qui est exprimée en utilisant des valeurs numériques (valeurs L*, valeurs a* et valeurs b*) dans les axes de coordonnées dans l'espace colorimétrique L*a*b*,

$$\text{Équation (1)} \quad \Delta E = \{(\Delta L^*)^2 + (\Delta a^*)^2 + (\Delta b^*)^2\}^{1/2}$$

Dans lequel, dans l'équation (1), $\Delta L^*$ est représenté par la différence entre la luminosité L* d'une première couleur et la luminosité L* d'une seconde couleur, $\Delta a^*$ est représenté par la différence entre la chromaticité a* de la première couleur et la chromaticité a* de la seconde couleur, et $\Delta b^*$ est représenté par la différence entre la chromaticité b* de la première couleur et la chromaticité b* de la seconde couleur, **caractérisé en ce que** le corps en colonne est formé d'une seule couche n'ayant pas d'interface stratifiée.

2. Ébauche de fraise pour découpe dentaire selon la revendication 1, dans laquelle, l'épaisseur dans le sens de la hauteur de chacune des « unités de couleur » étant définie comme une hauteur de chacune des « unités de couleur », les hauteurs de « l'unité de couleur 1 » (19) et de « l'unité de couleur m » (14) représentent chacune 15 à 35 % d'une hauteur totale du corps en colonne, et la hauteur de l'« unité de couleur 1 » et la hauteur de l'« unité de couleur m » sont facultativement différentes.

3. Procédé de production de l'ébauche de fraise pour découpe dentaire selon la revendication 1 ou 2, le procédé comprenant :

une étape de préparation de matière première consistant à préparer une composition de matière première de résine hybride (40) sous forme de pâte contenant un monomère polymérisable, une charge inorganique, un initiateur de polymérisation thermique et un agent colorant ;

une étape de remplissage consistant à mettre la composition de matière première de résine hybride dans un moule (30) correspondant à la forme de la section à découper (1) et

une étape de durcissement consistant à durcir la composition de matière première de résine hybride mise dans le moule par polymérisation thermique,

l'étape de préparation de la matière première consistant à préparer, sous forme de composition de matière première de résine hybride, la composition de matière première d'« unité de couleur 1 (19)» A donnant une résine hybride de la couleur : A et la composition de matière première d'« unité de couleur m (16) » B donnant une résine hybride de la couleur : B ; et, en utilisant des cuves capables de contenir séparément la composition de matière première d'« unité de couleur l » A et la composition de matière première d'« unité de couleur m » B et un dispositif de décharge capable de mélanger uniformément, selon un rapport désiré, les deux types de compositions de matière première de résine hybride contenues dans les cuves et en déchargeant un mélange à partir d'un orifice de décharge, on mélange la composition de matière première d'« unité de couleur » A et la composition de matière première d'« unité de couleur m » B avec un rapport de mélange de A et B variant, pour préparer les (m-2) types de compositions de matière première de résine hybride de zone intermédiaire donnant les couleurs : de l'« unité de couleur 2 » à l'« unité de couleur (m-1) »,

l'étape de remplissage comprenant :

une étape de remplissage de la zone de pointe consistant à extruder la composition de matière première d'« unité de couleur 1 » A à partir de l'orifice de décharge (26) pour mettre, dans le moule, une quantité nécessaire de la composition de matière première d'« unité de couleur 1 » afin de former l' « unité de couleur 1 » ;

une étape de remplissage de la zone d'extrémité de base consistant à extruder la composition de matière première d'« unité de couleur m » B à partir de l'orifice de décharge pour mettre, dans le moule, une quantité nécessaire de la composition de matière première d'« unité de couleur m » B afin de former l' « unité de couleur m » ; et

une étape de remplissage de zone intermédiaire consistant à extruder les compositions de matière première de résine hybride de zone intermédiaire préparées à l'aide du dispositif de décharge à partir de l'orifice de décharge pour mettre, dans le moule, les quantités nécessaires des compositions de matières premières de résine hybride de la zone intermédiaire afin de former l'« unité de couleur 2 (18) » à l'« unité de couleur m-1 (15) »,

l'étape de remplissage étant effectuée suivant l'un ou l'autre des modèles suivants :

1) un modèle 1 dans lequel l'étape de remplissage de la zone de pointe, l'étape de remplissage de la zone intermédiaire et l'étape de remplissage de la zone d'extrémité de base sont effectuées dans l'ordre mentionné ; et

2) un modèle 2 dans lequel l'étape de remplissage de la zone d'extrémité de base, l'étape de remplissage de la zone intermédiaire et l'étape de remplissage de la zone de pointe sont effectuées dans l'ordre mentionné, et

dans le modèle 1 et le modèle 2, les étapes de remplissage étant effectuées par extrusion continue de la composition de matière première de résine hybride tout en faisant varier, par étapes, la constitution de la composition de matière première de résine hybride qui doit être extrudée à partir de l'orifice de décharge.

4. Procédé de fabrication de l'ébauche de fraise pour découpe dentaire selon la revendication 3,

dans lequel, lorsqu'une position dans le moule est exprimée par un système de coordonnées dont l'origine est un point donné sur une surface inférieure du moule et dont l'axe x et l'axe y représentent respectivement une direction latérale et une direction longitudinale de la surface inférieure et dont l'axe z représente un sens de la hauteur, et (m-1) éléments de coordonnées z correspondant aux hauteurs des limites entre m éléments des « unités de couleur » qui doivent être formés dans le moule sont désignés par $z_1$ à $z_{m-1}$ du côté de la surface inférieure,

la mise de chacun des types de compositions de matière première d'« unité de couleur » dans le moule dans l'étape de remplissage est effectuée à l'aide du dispositif de décharge capable de commander librement une position de l'orifice de décharge (26) dans une direction avant-arrière, une direction gauche-droite, et le sens de la hauteur, en :

(i) réglant la position de l'orifice de décharge au début du remplissage, à l'origine ;

(ii) déplaçant la position de l'orifice de décharge dans la direction de l'axe x et/ou de l'axe y pour balayer une zone entière sur la surface inférieure une fois tout en déchargeant la composition de matière première de résine hybride avec un débit prédéterminé à partir de l'orifice de décharge, afin de former une zone de remplissage la plus basse du moule ayant une épaisseur uniforme correspondant au balayage ;

(iii) déplaçant la position de l'orifice de décharge dans la direction de l'axe z à une hauteur d'une surface supérieure de la zone de remplissage la plus basse du moule ou plus haut après avoir terminé le balayage en (ii) ;

(iv) déplaçant la position de l'orifice de décharge dans la direction de l'axe x et/ou de l'axe y pour balayer une zone entière sur la surface supérieure de la zone de remplissage la plus basse du moule servant de fondation une fois tout en déchargeant la composition de matière première de résine hybride avec un débit prédéterminé de la même manière que dans (ii), pour former une zone de remplissage développée résultant de l'addition d'une épaisseur uniforme correspondant au balayage ; et

(v) répétant les opérations (iii) et (iv) en continu avec la zone de remplissage développée servant de fondation, et

la variation du rapport dans la composition de matière première de résine hybride à décharger étant effectuée dans les opérations (ii) à (iv) de telle sorte que, à un moment où l'épaisseur de la zone de remplissage développée formée devient égale ou sensiblement égale à chacune des hauteurs $z_1$ à $z_{m-1}$, la composition de matière première pour l'« unité de couleur » suivante est déchargée depuis l'orifice de décharge.

5. Procédé de fabrication de l'ébauche de fraise pour découpe dentaire selon la revendication 3 ou 4,

dans lequel l'étape de remplissage effectue alternativement le remplissage de la composition de matière première de résine hybride dans le moule selon le modèle 1 et le remplissage de la composition de matière première de résine hybride dans le moule selon le modèle 2.

**FIG. 1**

**FIG. 2**

XY surface (Bottom surface)

(i) ⇒ (ii)

# FIG. 3

(i) ⇒ (ii)　　　　(iii) ⇒ (iv)

# FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016535610 A
- JP 2020151339 A
- US 2019247168
- JP 6285909 B
- JP 6349480 B
- WO 2018074605 A1